# NEW EUROPEAN PATENT SPECIFICATION

(11) **EP 1 187 840 B2**
(45) Date of publication and mention of the opposition decision: **19.12.2012**
(45) Mention of the grant of the patent: 10.06.2009
(21) Application number: 00936329.2
(22) Date of filing: 26.05.2000
(51) Int. Cl.: C12N 15/10

(54) **METHODS OF DNA PURIFICATION**
METHODEN ZUR REINIGUNG VON DNS
PROCEDES DE PURIFICATION D'ADN

(30) Priority: 28.05.1999 US 136772 P
(43) Date of publication of application: 20.03.2002
(73) Proprietor: Lonza Biologics Inc., Portsmouth, NH 03801 (US)
(72) Inventor: RAMASUBRAMANYAN, Natarajan, Baltimore, MD 21224 (US)
(74) Representative: Winter, Brandl, Fürniss, Hübner, Röss, Kaiser, Polte - Partnerschaft
(86) International application number: PCT/US2000/014527
(87) International publication number: WO 2000/073318

(56) References cited:
- EP-A- 0 964 057
- WO-A1-99/29832
- US-A- 5 622 960
- PRAZERES D M F ET AL: "Large-scale production of pharmaceutical-grade plasmid DNA for gene therapy: problems and bottlenecks" TRENDS IN BIOTECHNOLOGY, ELSEVIER, AMSTERDAM, NL, vol. 17, no. 4, April 1999 (1999-04), pages 169-174, XP004162836 ISSN: 0167-7799
- MCLAUGHLIN L W: "Resolution of RNA using high-performance liquid chromatography" JOURNAL OF CHROMATOGRAPHY, ELSEVIER SCIENCE PUBLISHERS B.V. AMSTERDAM, NL, vol. 418, 1987, pages 51-72, XP002112449 ISSN: 0021-9673
- FERREIRA G N M ET AL: "Downstream processing of plasmid DNA for gene therapy and DNA vaccine applications" TRENDS IN BIOTECHNOLOGY, ELSEVIER, AMSTERDAM, NL, vol. 18, no. 9, 1 September 2000 (2000-09-01), pages 380-388, XP004214265 ISSN: 0167-7799
- DIOGO M M ET AL: "Purification of a cystic fibrosis plasmid vector for gene therapy using hydrophobic interaction chromatography" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 68, no. 5, 5 June 2000 (2000-06-05), pages 576-583, XP002178573 ISSN: 0006-3592
- COLOTE ET AL.: 'Analysis and purification of plasmid DNA by reversed-phase high-performance liquid chromatography' ANALYTICAL BIOCHEMISTRY, vol. 154, no. 1, April 1986, pages 15 - 20, XP002930500
- DATABASE HCAPLUS, ACCESSION NO. 1999:688475 DIOGO ET AL.: 'Separation and analysis of plasmid denatured forms using hydrophobic interaction chromatography' & NALYTICAL BIOCHEMISTRY, vol. 275, no. 1, 1999, pages 122 - 124
- ONISHI ET AL.: 'An assay method for DNA topoisomerase activity based on separation of relaxed DNA from supercoiled DNA using high-performance liquid chromatography' ANALYTICAL BIOCHEMISTRY, vol. 210, no. 1, April 1993, pages 63 - 68, XP002930814
- GREEN ET AL.: 'Purification of nucleic acid-based pharmaceuticals with polyflo(TM) resin' CLINICAL CHEMISTRY, vol. 40, no. 12, 17 December 1994 - 19 December 1994, page 2335, XP002930815
- WEINER ET AL.: 'Plasmid purification using reverse-phase high performance liquid chromatography resin PRP' NUCLEIC ACIDS RESEARCH, vol. 16, no. 16, 25 August 1988, page 8185, XP002930816

## Description

The present invention provides methods for the purification of plasmid DNA that includes removal of host cell impurities, such as endotoxins, RNA , proteins and host cell DNA, from an aqueous solution containing plasmid DNA and methods for separation and purification of supercoiled plasmid DNA from an aqueous solution containing a mixture of supercoiled and nicked or relaxed plasmid DNA using hydrophobic interaction media. Also provided are improved methods for small-scale, such as laboratory or bench-scale, purification of DNA and equipment used in such methods. The present invention provides purified and/or separated plasmid DNA, such as supercoiled plasmid DNA.

Gene therapy offers a new treatment paradigm for curing human disease. The use of DNA for treatment of genetically caused diseases, including cystic fibrosis, various types of cancer, etc., and immunization against diseases, is a promising mode of therapy that is currently being widely pursued. Rather than altering the disease phenotype by using agents which interact with gene products, or are themselves gene products, gene therapy can theoretically modify specific genes resulting in disease cure or treatment. Gene therapy could also be used as a drug delivery system. To accomplish this, a gene that produces a useful product (RNA, peptide, protein, etc.) or is itself a useful product (such as in the use of antisense DNA) would be inserted into the DNA or cell of a homologous, heterologous or autologous cell of an individual or host cell to be later administered to an individual, either *in vivo*, *ex* vivo or *in vitro.* For example, during blood vessel surgery, a gene that makes an anticlotting factor could be inserted into the DNA of cells lining blood vessels to help prevent dangerous blood clots from forming. Many other conditions might also lend themselves to treatment using this general approach.

Gene therapy is expected to be a powerful tool for treating many of the more than 4,000 known genetic disorders, including cystic fibrosis, heart disease, cancer, arthritis, and other illnesses. Gene therapy generally requires the transfer of genetic material (DNA) into an individual. Gene delivery or the delivery of genetic material can be achieved either by direct administration of gene containing viruses or plasmid DNA to blood or tissues, indirectly through the introduction of cells manipulated in the laboratory to harbor foreign DNA or through various encapsulation or carrier techniques known in the art. Recent reports suggest direct delivery of DNA may also be possible. Several different systems are in use or under consideration for somatic gene transfer. These include, for example, DNA (either naked or complexed), RNA viruses (retroviruses), and DNA viruses (adenovirus, adenoassociated virus [AAV], herpes virus, and poxvirus).

The key advantages of non-viral mode of gene therapy, such as the use of plasmid DNA, is the ease of preparation in large quantities, a great degree of safety, a general lack of integration of the heterologous DNA into the host cell DNA, and the possibility of using gene(s) or gene(s) fragments of virtually unlimited size and number. Further, the use of plasmid DNA in gene therapy does not generally involve the use of extraneous gene(s) or proteins which may induce an unwanted immune response in the recipient. In addition, alternatives to the use of plasmid DNA, such as viral vectors, are relatively more expensive to produce.

Methods currently used to produce plasmid DNA generally provide a mixture of supercoiled plasmid DNA and a nicked (or relaxed) DNA artifact, which is generally not useful in the final application of the plasmid DNA. The methods of the present invention provide a non-destructive separation of supercoiled and nicked plasmid DNA such that while the present methods are exemplified by recovery and use of the supercoiled plasmid DNA, one of ordinary skill will appreciate that either separated form of the plasmid DNA may be considered a useful product of the presently disclosed methods. Moreover, while the present disclosure emphasizes the need for greater purity of supercoiled plasmid DNA in the context of gene therapy, one of ordinary skill in the art will appreciate that plasmid DNA is widely used in recombinant molecular biology beyond the use in gene therapy preparations and the presently disclosed invention finds wide applicability as a preparative method for isolated and purified supercoiled plasmid DNA.

Currently available methods for separation of the two forms of plasmid DNA utilize ion exchange chromatography (A novel, rapid process for purification of plasmids for gene therapy (Bhikhabhai R. Ollivier M. and Blanche F., Amersham Pharmacia Biotech R & D, 75184 Uppsala, Sweeden and RPR Gencell, Rhone-Poulenc Roer, Center de Recherche de Vitry-Alfortville, 13 quai Jules Guesde, 94400 Vitry sur Siene, France. Publication number: 18-1129-51; Preparative purification of supercoiled plasmid DNA using anion exchange chromatography, Duarte Miguel Prazeres, Thomas Schleup, Charles Cooney, Journal of Chromatography A, 606 (1998), 31-45) or size exclusion chromatography (Prazeres, D.M., A comparison of Gel Filtration Chromatographic Supports for Plasmid Purification, Biotechnology Techniques Vol. 11, No. 6, June 1997, p 417-420), coupled with the use of additives such as polyethylene glycol (PEG), detergents, and other components such as hexamine cobalt, spermidine, and polyvinylpyrollidone (PVP). Recently a patent was awarded (Horn, et al (U.S. Patent No. 5,707,812 )) for the purification of supercoiled plasmid DNA using PEG as an additive. However, currently known methods are unable to provide an efficient and cost effective separation of supercoiled and nicked (or relaxed) DNA. In addition, many of the known methods suffer from the disadvantage of using PEG or other additives, which may not be desired in manufacture of plasmid DNA, as they require additional separation, disposal and quality control methods, which can be difficult, more time consuming and more expensive.

Alternative forms of known methods for separation of supercoiled and relaxed forms of plasmid DNA utilize very expensive, proprietary resins, which also utilize solvents, such as acetonitrile, ethanol and other components, like triethylamine and tetrabutyl ammonium phosphate, during processing. These methods are generally not suited for large-scale production due to the use of solvents. Moreover, they cannot be applied to starting materials that have significant amount of relaxed Plasmid DNA as the abundant amount of contaminating relaxed plasmid DNA in starting materials tends to reduce the resolution capabilities of these resins. (Green, A. P. et al. Bio. Pharm. Vol. 10, No. 5, pages 52-62, May 1997.)

Additional methods of separating supercoiled and relaxed DNA rely on size-exclusion chromatography, which involves separation of the two forms of plasmid DNA based on the small difference in size. These columns tend to be relatively long, posing significant scale-up problems, making it infeasible to implement in large-scale production. In addition size-exclusion methods need concentrated sample solutions, that are infeasible to obtain with plasmid DNA solutions, due to the highly viscous nature of the DNA. See, A comparison of gel filtration chromatographic supports for plasmid purification G.N.M. Ferreira, J.M.S. Cabral and D.M.F. Prazeres, Biotechnology Techniques, Volume 11, No. 6, June 1997, pp 417-420.

Plasmid DNA preparations, which are produced from bacterial preparations and often contain a mixture of relaxed and supercoiled plasmid DNA, often requires endotoxin removal, as required by the FDA, as endotoxins produced by many bacterial hosts are known to cause inflammatory reactions, such as fever or sepsis in the host receiving the plasmid DNA. These endotoxins are generally lipopolysacchrides, or fragments thereof, that are components of the outer membrane of Gram-negative bacteria, and are present in the DNA preparation as artifacts of the hosts cells or as a part of larger artifacts, such as host cell membranes or macromolecules, used in expression and manufacture of the plasmid DNA for gene therapy, for example. Hence removal of endotoxins is a crucial and necessary step in the purification of plasmid DNA for therapeutic or prophylactic use.

Endotoxin removal from plasmid DNA solutions primarily have used the negatively charged structure of the endotoxins; however plasmid DNA also is negatively charged and hence separation is usually achieved with anion exchange resins which bind both these molecules and, under certain conditions, preferentially elute plasmid DNA while binding the endotoxins. Such a separation results in only partial removal as significant amounts of endotoxins elute with the plasmid DNA and/or a very poor recovery of plasmid DNA is achieved. Other patented methods use detergents, which could pose problems. (Process for the depletion or removal of endotoxins. Coplan, Metin, Moritz, Peter, Schorr, Joachim, U.S. Patent Number: 5,747,663.) In addition, the binding capacity of these resins is only on the order of 10³ to 10⁴ EU (endotoxin units) /ml of resin as the resin is occupied by both endotoxin and plasmid DNA, for example, typically requiring 3 to 80 liters of resin, based on reported capacities of 50,000 EU/ml to 2000 EU/ml (Green, A. P. et al. Bio. Pharm. Vol. 10, No. 5, pages 52-62, May 1997. Sterogene technical profile DNA Etox, Sterogene, 5922 Famsworth Cr., Carlsbad, CA 92008).

Prazeres et al., Trends in Biotech. 17(4):169-174 (1999) discusses the problems associated with large scale production of pharmaceutical-grade plasmid DNA for gene therapy and notes that the low capacity of anion-exchange chromatographic sorbents is a pertinent issue in plasmid purification.

McLaughlin et al., J. Chromatog. 418:51-72 (1987) describes methods for the resolution of RNA using HPLC. in particular anion-exchange and reversed-phase chromatography.

WO 99/29832 describes an HPLC chromatographic method for purifying plasmid DNA from a mixture containing both plasmid and genomic DNA. Ethanol is used as a solvent for eluting plasmid DNA.

Colote et al., Analytical Biochem. 154:15-20 (1986) describes a method for the analysis and purification of plasmid DNA by reversed-phase high-performance liquid chromatography. The process described is carried out in the presence of organic solvents such as acetonitrile and DMSO.

The present invention provides methods of plasmid DNA separation, isolation and/or purification which may be used in combination or independently. Specifically, the present invention provides methods of separation, purification and/or isolation of supercoiled and relaxed plasmid DNA as well as methods of separation, purification and/or isolation of plasmid DNA from host cell impurities, such as endotoxin containing components or fragments. The present invention also provides methods for laboratory- or bench-scale separation, isolation and/or purification of plasmid DNA.

The present invention provides methods for isolating plasmid DNA from other components present in mixtures containing plasmid DNA, yielding compositions enriched in the desired plasmid DNA. The methods include the separation of the plasmid DNA from the undesired components by contacting mixtures containing the plasmid DNA with hydrophobic interactive media. The separation of the plasmid DNA from other components results from the differing affinities of the plasmid DNA and other undesired components for the hydrophobic interactive media under differing ionic conditions. Thus, in the methods of the invention a hydrophobic interactive media is used that has a highly preferential binding for lipopolysaccharides and lipoproteins relative to plasmid DNA; this preferential binding occurs over the range of ionic conditions used in the separation process. Also included in the invention are methods that separate supercoiled plasmid DNA and relaxed plasmid DNA. The methods utilize ionic conditions wherein the supercoiled plasmid DNA binds preferentially to the hydrophobic interactive media relative to the relaxed plasmid DNA.

It is understood that, when describing a salt concentration used in the methods of this invention, that an equivalent ionic strength of a different salt may be used.

It is an object of the present invention to provide methods of plasmid DNA separation, isolation and/or purification from contaminating host cell impurities.

It is another object of the present invention to provide methods of separation, purification and/or isolation of plasmid DNA and endotoxin containing components or fragments.

It is yet another object of the present invention to provide methods of separation, purification and/or isolation of supercoiled and relaxed plasmid DNA.

In one embodiment, the present invention provides a method of separating endotoxin and other host cell impurities (i.e., RNA, chromosomal DNA, protein) from plasmid DNA involving contacting a cell lysate with a hydrophobic interaction media under conditions where the endotoxin and other contaminating substances bind to the hydrophobic interaction media to form a complex and separating the plasmid DNA and the complex. The endotoxin separated in the method of this embodiment includes endotoxin from Gram-negative microorganisms as well as fragments and cellular and subcellular components bound to these endotoxins and endotoxin fragments. The hydrophobic interaction medium also binds RNA, including t-RNA, r-RNA and m-RNA, host cell proteins and chromosomal DNA. The hydrophobic interaction media useful in the present invention may be in the form of resins, membranes or other support media.

A preferred form of this embodiment includes loading of the mixture of plasmid DNA, with optional other contaminating host cell components, including endotoxin, being present, on a column or bed matrix containing the hydrophobic interaction media, in a manner where the endotoxin and host cell impurities preferentially binds or is retained by the hydrophobic interaction media, and the plasmid DNA is collected as effluent (flow-through) from the loading process or in optional subsequent washing(s) of the hydrophobic interaction media which do not disturb or disrupt the retention of the host cell impurities and endotoxin on and/or in the hydrophobic interaction media. After collection of the plasmid DNA, the column or bed matrix may be regenerated by eluting bound or retained host cell impurities and endotoxin by altering, changing or modifying the hydrophobic interaction conditions of the column or bed by, for example, altering the salt concentration surrounding the column or bed matrix.

Alternate embodiments of the present invention provide methods of separation in the absence of either or both ion exchange chromatography or size exclusion chromatography.

In one preferred form of this embodiment, the column or bed volume is initially equilibrated with an ammonium sulfate reaction solution at a concentration which allows selective binding of the contaminating impurities to the hydrophobic interaction column, preferably, a concentration of about 2M. Salts which may be used in the method of the present invention include mixtures of anions and cations selected from the group consisting of, but not limited to, acetate, phosphate, carbonate, SO₄²⁻ . Cl⁻, Br⁻ , NO₃⁻, Mg²⁺. Li⁺, Na⁺, K⁺, NH₄⁺. Mixtures of salts may be used. Moreover, the mixture of plasmid DNA and other contaminating impurities, such as endotoxin, are preferably dialyzed with a dialysis buffer prior to contacting with the column or bed matrix to remove salts and other contaminants which may alter the hydrophobicity of the endotoxin and plasmid DNA and other contaminating impurities, such as endotoxin, in the ammonium sulfate reaction solution. The reaction solution is preferably buffered with, for example, Tris-HCl at a pH in the range of, but not limited to, 6.8 to 8.5, preferably 7.4. Other buffers, are known to those skilled in the art, such as, but not limited to, Tris, TES (N-tris(hydroxymethyl)methyl-2-aminoethane-sulfonic acid), Tricine (N-tris(hydroxymethyl) methylglycine), phosphate, PIPES (Piperazine-N,N'-bis(2-ethane sulfonic acid), MOPS (3-(N-morpholino)-propanesulfonic acid), MES (2-(N-morpholino)-ethanesulfonic acid), MEPES (3-N(N-Morpholino)ethylpiperazine-N'-2-ethanesulfonic acid), and Bicine (N,N-bis(2-hyroxyethyl) glycine) may be used.

The methods of the present invention provide isolated and/or purified plasmid DNA, and a composition containing the plasmid DNA, which has a substantially-reduced endotoxin content, or is endotoxin-free, such that the endotoxin load of the plasmid DNA has been reduced by as much as greater than 95%, preferably, greater than 98%, more preferably greater than 99%, alternatively greater than 99.9% and most preferably greater than 99.99% to 99.999%. The method of the present invention may be used to separate large initial loads of endotoxin, such as at least 200,000 to 400,000 endotoxin units (EU)/milliliter of solution, providing a capacity of at least 600,000 to 3 million EU/ml hydrophobic matrix. Moreover, the process of the present invention provides a product plasmid DNA composition containing a range of less than 10 to less than 2500 EU or a range of less than 1 to less than 300 EU/mg DNA. Alternatively, the process of the present invention provides a product plasmid DNA composition containing a range of less than 50 to less than 1000 endotoxin units (EU) or a range of less than 10 to less than 50 EU/mg DNA. The method of the present invention also reduces protein content to less than 0.1% (w/w), RNA to less than 1% (w/w) and chromosomal DNA to less than 1% (w/w).

In another embodiment, the present invention provides a method of separating supercoiled plasmid DNA from relaxed plasmid DNA which includes the steps of contacting a mixture of supercoiled plasmid DNA and relaxed plasmid DNA with a hydrophobic interaction media under a first condition where both the supercoiled plasmid DNA and the relaxed plasmid DNA bind to the hydrophobic interaction media to form a bound first mixture, altering the first conditions surrounding the bound first mixture to a second condition to remove the relaxed plasmid DNA from the bound first mixture to form separate components containing a second bound mixture and the relaxed plasmid DNA, and modifying the second conditions surrounding the second bound mixture to a third condition to remove the supercoiled plasmid DNA from the second bound mixture to form separate components containing the hydrophobic interaction media and the supercoiled plasmid DNA.

In another form of this embodiment, the altering and modifying can be performed by changing the pH conditions of the eluting buffer or solution, in such a way that the relaxed and supercoiled forms could be eluted at different pH conditions, with or without change in salt conditions.

In another form of this embodiment, the altering and modifying can be performed through isocratic elution, where a solution of the same composition, preferably at a salt concentration that can elute both forms of the plasmid DNA, when passed through the column containing the bound plasmid, would sequentially elute the two forms distinctly, in separate fractions.

In another embodiment, the salt conditions and/or other conditions can be modified in such a way that the relaxed form of the plasmid could be collected as the unbound fraction, while the supercoiled form binds to the resin. The supercoiled form can subsequently be eluted by using conditions outlined above.

In another form of this embodiment, the altering and modifying can be performed by use of molecules or a mixture of molecules that competitively bind to the ligands ("displacers") and remove the bound plasmid DNA forms from the matrix as separate components.

In another form of this embodiment, molecules or mixture of molecules that can bind through hydrophobic interaction or otherwise, can be mixed with plasmid DNA solutions, which, on loading on to the column could be sequentially displaced, resulting in separation of the different forms of DNA.

In the above two alternative forms of the embodiment, commonly referred to as "displacement" and "frontal" mode of chromatography, the added molecule may co-elute with the product which, in most cases can be effectively separated using methods known to those in the art.

Displacement chromatography is a mode of chromatography in which two or more molecules bound to a resin are displaced using a displacer molecule that has higher affinity for the resin resulting in sequential displacement and hence elution of the two or more bound molecules. Recently, displacers for hydrophobic interaction resins have been identified, which consists of triblock copolymers including polymethyl methacrylate, acrylic acid, and polydimethylaminoethyl methacrylate (see Ruaan et al "Hydrophobic displacement chromatography of proteins using triblock copolymers as displacers, 1998 AIChE meeting). Other displacers have been successfully developed for displacement chromatography with hydrophobic interaction resins (see Shukla et. al. Hydrophobic displacement chromatography of proteins in 1998 Annual AIChE meeting). Displacers such as 2-(2-butoxyethoxy)ethanol have been used as displacers in reverse phase chromatography, which might be useful.

After binding the two forms of the plasmid, a displacer, such as ones listed above could be used to displace supercoiled and relaxed DNA sequentially from the HIC (hydrophobic interaction column) resins described herein.

In "Frontal" mode of chromatography, the column is loaded with a binary mixture, differing in their affinity for the resin, and upon continually overloading the column, one component displaces the other and results in sequential elution of the two components. In the application of this method for the current invention, the two forms of DNA, for example, could be loaded on a HIC column and overloading of the sample could result in displacement effect leading to the displacement of the relaxed form, which can be collected separately from the supercoiled form.

In one form of this embodiment, the altering and modifying are combined in a continuous process of a gradient elution of the relaxed plasmid DNA and supercoiled plasmid DNA by mixing the bound first mixture with a salt solution, such as ammonium sulfate solution, with a continuously varying concentration of salt , such as ammonium sulfate, the concentration preferably varying from 3M to 1M salt, such as ammonium sulfate. The relaxed plasmid DNA is collected in this form of this embodiment of the invention in a first eluted volume and the supercoiled plasmid DNA is collected in a second eluted volume.

In another preferred form of this embodiment, the supercoiled and relaxed forms of the plasmid DNA are separated by first binding both forms of the DNA to a hydrophobic interaction media in a bed or column at high salt concentrations, or equivalent ionic strengths, such as 2.5 M to 4 M, preferably 3 M, ammonium sulfate, and then eluting, either in a step gradient or continuous gradient manner, the two separate forms of the plasmid DNA off the column, by changing the salt concentration, or equivalent ionic strengths, to a first range of 2.45M to 2.35M ammonium sulfate and then to a second range of 0M (possibly 1M) to 2.3M ammonium sulfate (in the step elution embodiment) or by continuously changing the ammonium sulfate concentration from the range of 2.5 M to 4M to a second range of 0M (possibly 1M) to 2.3M over a volume of 1 to 30 column or bed volumes, preferably at least 6 column or bed volumes (in the continuous gradient embodiment). In each of these forms, the relaxed plasmid DNA elutes from the column or bed media at a salt (ammonium sulfate) concentration in the range of 2.35M to 2.45M whereas the supercoiled plasmid DNA elutes from the column or bed media at a salt (ammonium sulfate) concentration in the range of 0M to 2.3M.

In another embodiment, the invention provides methods of isolating supercoiled plasmid DNA which includes:
applying a sample containing supercoiled plasmid to a hydrophobic interaction media under ionic conditions whereby the supercoiled plasmid preferentially binds to the media with respect to non-supercoiled plasmid; and
adjusting the ionic conditions such that bound supercoiled plasmid is removed from the media.

In another preferred embodiment, the present invention provides a method for the enriching the amount of supercoiled plasmid DNA relative to relaxed plasmid DNA in a mixture thereof, the method including (1) interacting the mixture containing supercoiled plasmid DNA and relaxed plasmid DNA with a hydrophobic interactive media containing an alkyl moiety under ionic conditions wherein the supercoiled plasmid DNA preferentially binds to the hydrophobic interactive media; (2) treating the hydrophobic interactive media containing the relaxed and supercoiled plasmid DNA under ionic conditions that allow the preferential removal of the relaxed plasmid DNA; and (3) eluting the supercoiled plasmid DNA from the hydrophobic interactive media.

In a further preferred embodiment, the present invention provides a method for removing lipopolysaccharide (LPS) from a composition containing plasmid DNA, the method including the steps of (1) interacting the mixture containing the plasmid DNA and LPS with a hydrophobic interactive media containing an alkyl moiety, wherein the interacting is under ionic conditions where the LPS preferentially binds to the hydrophobic interactive media relative to the plasmid DNA; and (2) treating the hydrophobic interactive media containing the plasmid DNA and LPS with ionic conditions that allow the selective removal of the plasmid DNA.

The methods of the present invention provide isolated and/or purified supercoiled plasmid DNA, and a composition containing the supercoiled plasmid DNA, which is preferably endotoxin free, such that the amount of supercoiled plasmid DNA present in the composition produced by the presently disclosed methods is at least 50% by weight of the total plasmid amount to at least 99% by weight, preferably at least 60% by weight to at least 95% by weight, more preferably at least 70% by weight to at least 90% by weight, most preferably at least 75% by weight to at least 85% by weight, supercoiled plasmid DNA.

Weight percent may be measured, as exemplified herein, by HPLC resolution on a DNA-NPR HPLC column, through a gradient, resulting in peaks with areas corresponding to the amount of each component. The percentage of supercoiled form was calculated as the fraction of the peak area corresponding to supercoiled plasmid DNA to the total area of the supercoiled and relaxed plasmid DNA peaks.

Preferred hydrophobic interaction media which may be used in the methods of the present invention include hydrophobic interaction chromatography resins that, for example, contain methacrylate polymer or copolymer backbones, such as methacrylate /ethylene glycol and/or methacrylate/propylene glycol copolymers (TosoHaas, Montgomeryville. PA). and/or an agarose or Sepharose (Amersham Pharmacia Biotech. Piscataway, NJ), such as crosslinked or non-crosslinked, agarose, Sepharose, dextran, silica containing polymer, organic polymers (natural or synthetic), a ceramic-containing, or a gel matrix, backbone, or a combination of any of these, with C₃ to C₁₀ alkyl, branched or straight, pendent side chain ligands. Preferred pendent hydrophobic groups include propyl, butyl, hexyl and/or octyl ligands. These ligands provide the preferential binding interaction which is exploited in the separation, purification and/or isolation methods of the present invention. One of ordinary skill in the art will appreciate that hydrophobic interaction resins may include ligands in addition to or in place of these alkyl ligands, which will also be useful in the method of the present invention. Examples of such ligands include, but are not limited to, phenyl, octyl, butyl, propyl, neopentyl, hydroxypropyl, benzyl, octadecyl, diphenyl, and methyl as well as substituted and unsubstituted derivatives of same, and combinations thereof. Suitable resin or media materials useful in the present invention include those described, for example, in EP Patent No. 964057, EP Application No. 99109441, JP 2000035423, JP 99127700 and JP 98127665 (Kitamura et al.),

The hydrophobic interaction media may be in the form of beads, which may be packed or loaded into a column or bed reactor, or a crosslinked porous media. The size of bead media may range from 2.5 µm to greater than or equal to 100 µm. The size of bead media is preferably in the range of 30 to 110 µm in diameter, such as 35 to 100 µm in diameter, or, alternatively in the range of 35 to 90 µm in diameter. The hydrophobic interaction media may be present in the form of membranes, such as cellulose or cellulose derivative backbones, polyether sulfones, polysulfones, and derivatives of same and/or other materials known in the filtration and separation arts, including plastics, such as and including microtiter plates and petri or cell culture dishes and containers.

The beads used in "Streamline" (Amersham Pharmacia biotech) columns typically are larger in size with different densities, and are made by various manufactures, including (Amersham Pharmacia Biotech, Biosepra Inc, but not limited to these, where the clarified lysate could be flowed through these "expanded bed" columns, resulting in removal of contaminants through binding to the beads that contain the hydrophobic interactive ligands.

Smaller bead sizes, typically are used in high performance separations, including HPLC, where this invention can be utilized to provide a quantitative analytical method for plasmid DNA.

The beads for the purposes of use in this invention, particularly the separation of two forms of plasmid DNA does not need to be porous as the plasmid DNA is generally too large to be able to occupy the pores, and hence provide any additional capacity. However, for the purposes of contaminant binding, pores could effectively increase capacity as the contaminant molecules such as RNA, protein, endotoxin and DNA fragments are comparable or smaller then the size of pores. In this case porosity will play a role and hence porous resins may be useful.

In the methods of the present invention:
(i) purifying plasmid DNA from a mixture of the same containing at least one host cell impurity;
(ii) separating supercoiled plasmid DNA from relaxed plasmid DNA; and
(iii) enriching the amount of supercoiled plasmid DNA relative to relaxed plasmid DNA in a mixture thereof;
the method is conducted in the absence of organic solvents, detergents, glycols, hexamine cobalt, spermidine and polyvinylpyrrolidone, which would later require separation from the product supercoiled plasmid DNA prior to use in, for example, gene therapy.

### Brief Description of the Drawings

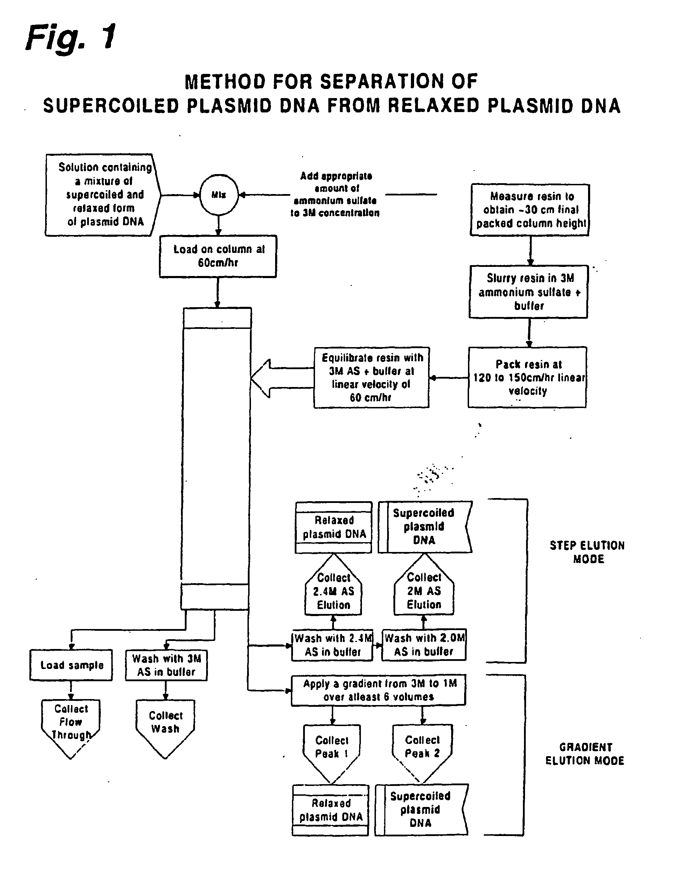

Figure 1 shows a flow diagram of the various steps involved in performing the exemplified method of separating supercoiled and relaxed plasmid DNA.

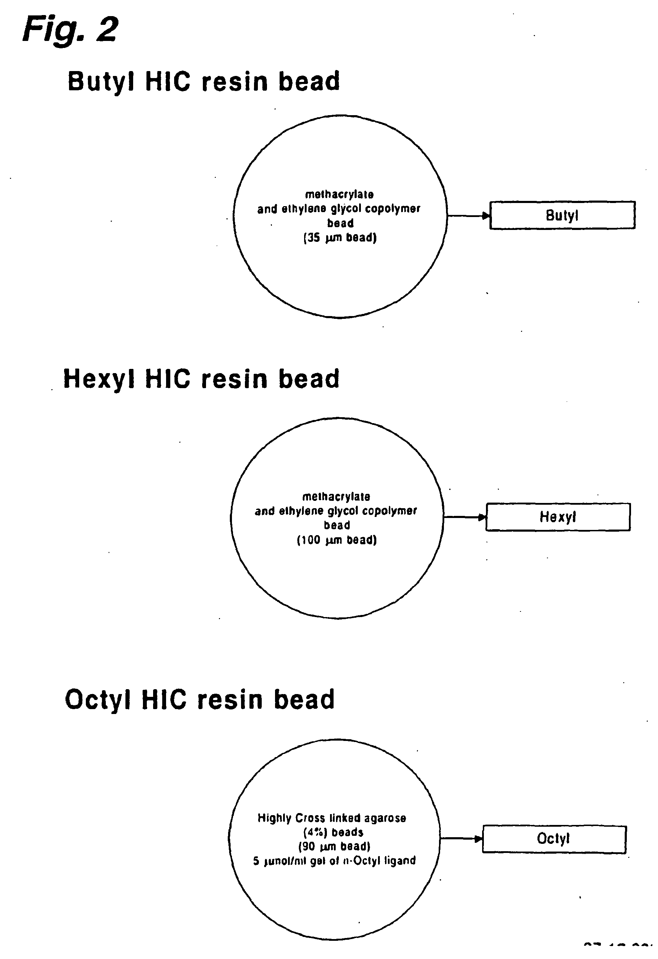

Figure 2 shows a conceptual depiction of the various hydrophobic interaction supports with the ligand chemistries attached to them that were used in the exemplified method.

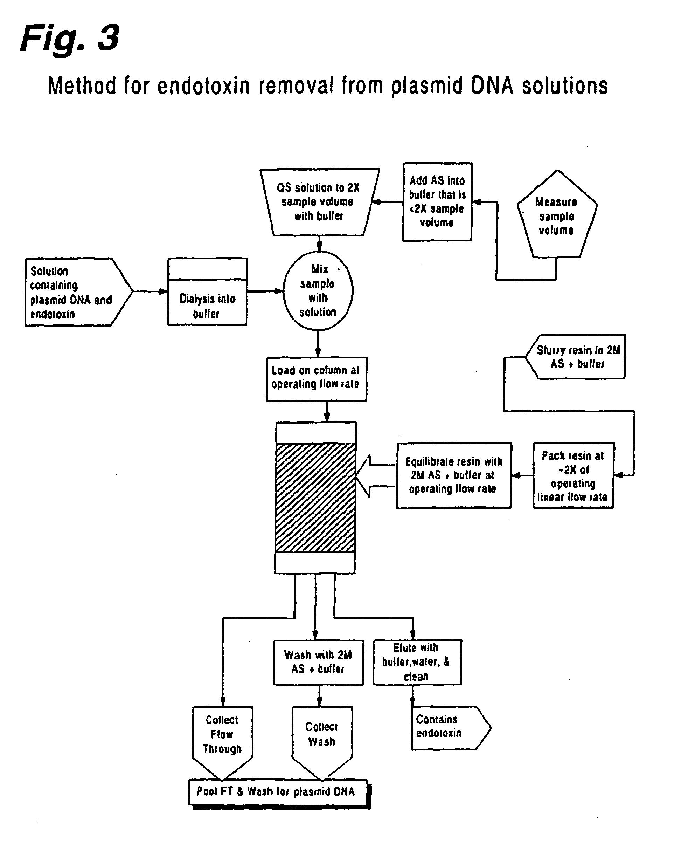

Figure 3 shows a flow diagram of the various steps involved in performing the exemplified method of separating endotoxin from plasmid DNA.

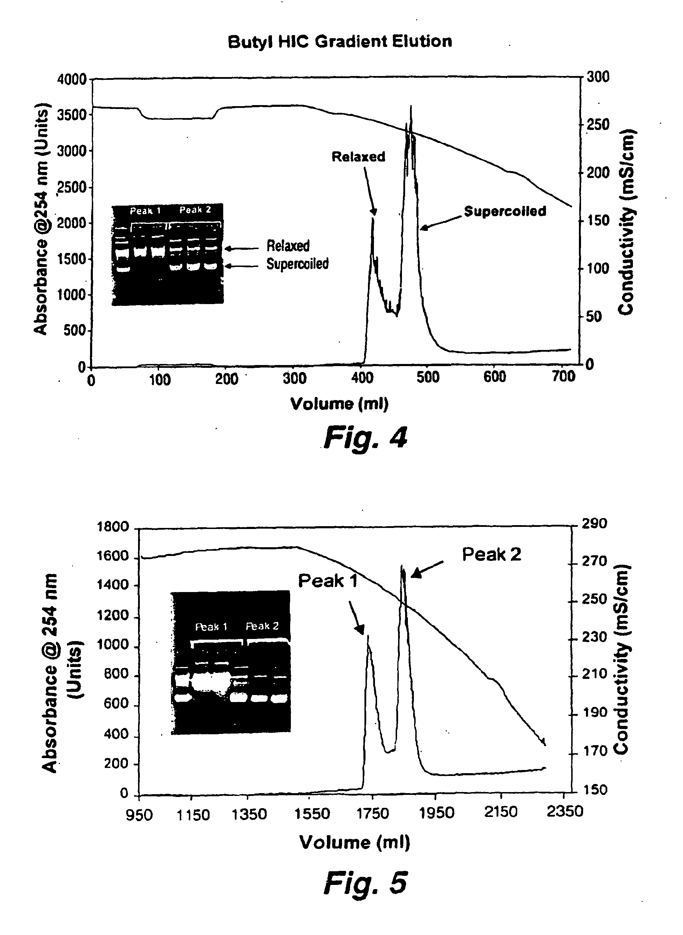

Figure 4 (insert) shows a scanned image of an agarose gel from Example 5 (butyl HIC) stained with SYBR GOLD wherein lane 1 contains a supercoiled DNA Ladder; lanes 2 and 3, contain samples from peak 1 (relaxed) and lanes 3-5 contain samples from peak 2 (supercoiled). (Lanes being numbered from left to right.) The chromatogram from Example 5 of absorbance versus volume shows the separation of relaxed (peak 1) and supercoiled DNA (peak 2).
Figure 5 (insert) shows a scanned image of an agarose gel from Example 6 (butyl HIC) stained with SYBR GOLD wherein lane 1 contains a marker; lanes 2 and 3 contain samples from peak 1 (relaxed) and lanes 4-6 contain fractions from peak 2 (supercoiled form). (Lanes being numbered from left to right.) The chromatogram from Example 6 shows the separation of relaxed (peak 1) and supercoiled DNA (peak 2) forms.

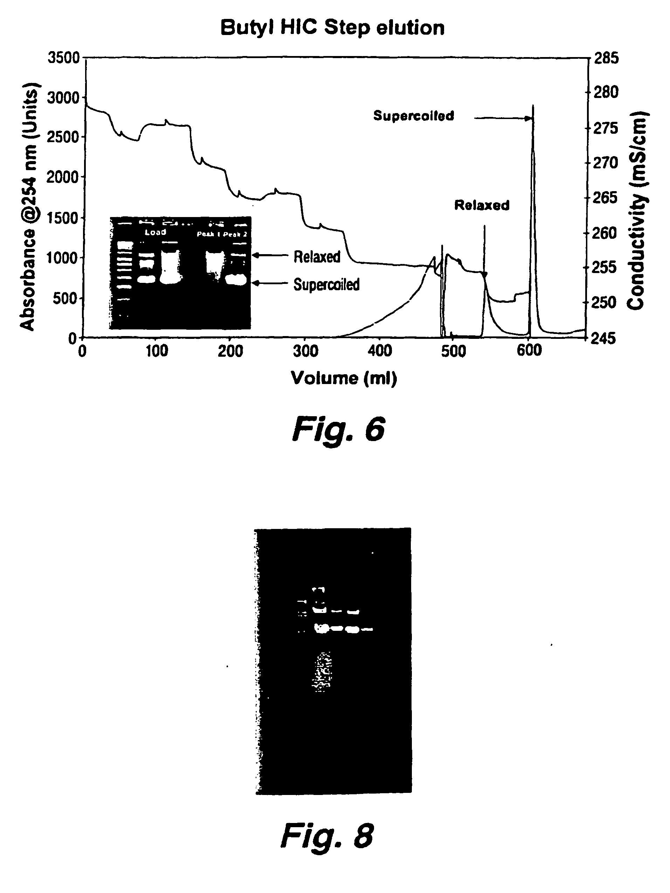

Figure 6 (insert) shows a scanned image of an agarose gel from Example 7 wherein lane 1 contains a marker, lanes 2 and 3 contain material loaded on the column; lane 4 contains material from the artifact peak; lane 5 contains material from the 2.4M AS Elution; and lane 6 contains material from the 1M AS Elution. (Lanes being numbered from left to right.) The chromatogram shown separation of relaxed and supercoiled forms of plasmid DNA wherein the artifact peak is a broad first (left) peak, followed to the right by relaxed and supercoiled peaks, respectively.

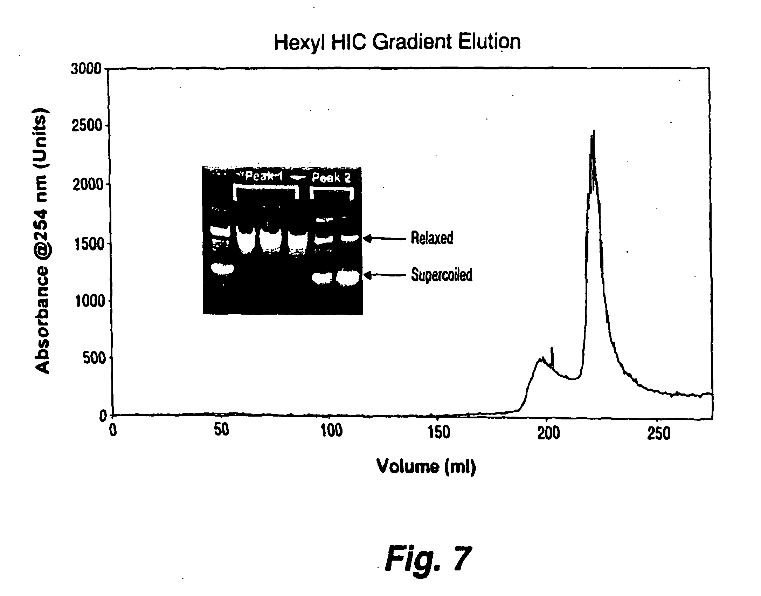

Figure 7 (insert) shows a scanned image of an agarose gel from Example 8 (hexyl HIC) wherein lane 1 contains a marker; lanes 2-4 contain material from peak 1 (relaxed form); and lanes 5 and 6 contain fractions from peak 2 (supercoiled form). (Lanes and peaks numbered from left to right.) The chromatogram demonstrates the separation of relaxed from supercoiled forms.
Figure 8 shows a scanned image of an agarose gel electrophoresis of samples SYBR GOLD stained, from Example 9, wherein lane 1 is a marker; lane 2 contains the material loaded on the column; lanes 3, 4 and 5 contain the washes 1, 2 and 3, respectively; lane 6 contains the 1M elution; and lane 7 contains the water elution.

The methods of the present invention exploit the differences in hydrophobicities of supercoiled plasmid DNA, relaxed plasmid DNA and cellular contaminants, such as endotoxin.

The methods disclosed herein are useful for purifying and isolating supercoiled plasmid DNA including cosmids and phagemid vectors. These vectors and plasmid DNA could be purified from any source. In addition, plasmid DNA and cosmids present in yeast and mammalian cells can also be purified, as similar mixtures of contaminants, including endotoxins, RNA, proteins and chromosomal DNA, could be present in these preparations. There is also the need to obtain supercoiled form of the plasmid and cosmid DNA from these sources and hence, the methods described herein could be used in purifying DNA in many of these applications.

"Hydrophobic interaction media" is a material comprising (a) a support moiety and (b) a hydrophobic moiety attached either directly or indirectly to the support moiety, examples of which are described herein and are known in the art. The hydrophobic moiety provides the basis for preferential binding used in the separation methods described herein. Various examples of "hydrophobic interaction media" are known in the art and are described herein. Other terms used herein also denote hydrophobic interaction media and examples thereof, such as "resin", "matrix", "column", "media", "beads" and "Hydrophobic interaction ligand".

"DNA" means any form of deoxyribonucleic acid, including, but not limited to, plasmid (whether supercoiled and/or relaxed or nicked), cosmid, or artificial chromosome.

"Nicked" or "relaxed" DNA means DNA that is not supercoiled. "Supercoiled" DNA is a term well understood in the art.

A "contaminating impurity" is any substance from which it is desired to separate, or isolate, DNA. Contaminating impurities include, but are not limited to, host cell proteins, endotoxin, host cell DNA and/or RNA. It is understood that, what is considered a contaminating impurity can depend on the context in which the methods of the invention are practiced. A "contaminating impurity" may or may not be host cell derived, i.e., it may or may not be a host cell impurity.

"Isolating" or "purifying" a first component (such as DNA) means enrichment of the first component from other components with which the first component is initially found. Extents of desired and/or obtainable purification are provided herein. Preferably, the methods of the invention result in an about five-fold enrichment, preferably an about 10-fold enrichment, preferably an about 20 fold enrichment, preferably an about 50 fold enrichment, preferably an about 100 fold enrichment, preferably an about 200 fold enrichment, preferably an about 500 fold enrichment, preferably an about 1000 fold enrichment. Alternatively, the degree of purification may be expressed as a percentage of the first component with respect to another component, or with respect to the resultant preparation. Examples of such percentages are provided herein.

"Preferential" or "selective" binding or removal of a component means that, for a given condition, the first component binds or is removed to a greater degree with respect to another component.

As would be understood by those skilled in the art, "removal" or "binding" does not necessarily, or even desireably, mean complete, or 100%, removal or binding.

An "aqueous" solution generally indicates a water-based solution (i.e., the main solvent is water), which may or may not be 100% water as solvent.

Isolation of plasmid DNA produced in recombinant bacterial cells involves lysis of the cells and removal of cellular debris, which can be accomplished through various methods. The final solution contains plasmid DNA, typically containing extremely high amounts of endotoxins among other contaminants. The methods of the present invention provides for the removal of significant amounts of the key contaminants, such as RNA, genomic DNA, protein and endotoxin using hydrophobic interaction chromatography as a first step, where the plasmid DNA flows through unbound. A method of the present invention involves, optionally dialyzing the mixed solution obtained from bacterial lysis into a buffer in the pH range of 6.8 to 8.5, preferably a pH of 6.8 to 7.4, containing, preferably, 2M ammonium sulfate, with or without 10mM ethylenediaminetetraaceticacid (EDTA), and flowing the optionally dialyzed solution through a packed chromatography column containing a chromatographic support with a hydrophobic interaction ligand(s), such as any of, or a mixture of, a propyl, a butyl, octyl or hexyl ligand, which had, preferably previously been equilibrated with a buffer in the pH range of 6.8 to 8.5 (preferably 6.8 to 7.4), also containing 2M ammonium sulfate, with or without 10mM EDTA. The flow-through solution is typically at about a 2M concentration of salts, such as ammonium sulfate, and predominantly contains plasmid DNA (mixture of supercoiled and relaxed) with less than 2% of contaminants. Depending on the variations in the upstream steps, the percentage of supercoiled DNA can range anywhere between 50 and 95% by weight, typically about 75 to 85%, alternatively, 80 to 85%, requiring further purification, involving the removal of the relaxed form of plasmid DNA from the mixture.

Methods of the present invention also make it possible to produce purified plasmid DNA which have endotoxin levels below specified levels, i.e., typically <10 EU/mg plasmid DNA. The methods of endotoxin removal of the present invention are either adaptable to large-scale or small-scale production, enabling economical production of therapeutic and laboratory grade material. These methods exploit the selective binding of endotoxin to the hydrophobic resins described herein which, for large-scale production, may contain capacities exceeding one million units per milliliter of resin used.

Conventionally available methods of endotoxin removal have low capacities (1,000 to 50.000 EU/milliliter of resin) and/or result in low plasmid DNA recoveries and/or involve use of chemical components and/or methods that cannot be readily used in preparation of therapeutic grade material. (U.S. Patent No. 5,747,663) The method of endotoxin removal of the present invention involves suspending the endotoxin- and plasmid DNA-containing solution in a salt, such as ammonium sulfate or sodium chloride at a concentration of 2M which makes the endotoxin significantly more hydrophobic than the plasmid DNA and assures binding or preferential interaction and separation of the endotoxin on a resin containing hydrophobic interaction ligands, such as butyl, octyl, and/or hexyl groups, as compared with the plasmid DNA. The salt concentration used may preferably be optimized to bind RNA, protein and endotoxin. A lower salt concentration may be sufficient to provide endotoxin binding alone as endotoxin has a greater affinity for the hydrophobic interaction ligands described herein.

An attractive feature of this method of endotoxin removal is the immense capacity of the resin for the endotoxin, of approximately 1,000,000 EU/ml of resin, in addition to the simplicity and >95% recovery of plasmid DNA. For example, a plasmid DNA solution containing 500 mg of plasmid and 10 million EU of endotoxin can be purified using 10 ml of resin, whereas, at least 1,000 to 4,000 ml of an anion exchange resin would be required for binding the plasmid DNA and the endotoxin, with the added disadvantage of poor recoveries on such an anion exchange resin. The method of the present invention therefore results in savings of 100 to 400 fold in resin cost, and additional savings on column cost and increased recovery of product. The commercially available DNA Etox resin is currently at least 8 fold more expensive than the resins used in the method of the present invention. Another commercial resin (PolyFlo - PureSyn Inc., 87 Great Valley Pkwy Malvem, PA 19355) with proprietary chemistry that is useful in endotoxin removal is 5 to 10 fold more expensive and requires the use of solvents and ion-pairing chemicals.

The methods of the present invention provide high quality plasmid DNA comprising greater than 90% supercoiled plasmid DNA from starting material of lesser quality (i.e., a starting material composed of a mixture of relaxed and supercoiled DNA). Additionally, the methods of the present invention are applicable for large-scale processes typically used for production of plasmid DNA for gene therapy. The methods of the present invention enable reliable production of high quality plasmid DNA, independent of variations that typically lead to reduction in quality i.e. generation of relaxed/nicked form of plasmid DNA. These variations could occur during growth of the bacteria producing the plasmid DNA and subsequent isolation and purification steps.

The methods of separating supercoiled and relaxed plasmid DNA, and methods of separating plasmid DNA and endotoxin, of the present invention are based on a discovery that the forms of plasmid DNA and endotoxin exhibit different binding specificities on hydrophobic interaction chromatography resins that, for example, contain C₄ to C₁₀ alkyl, branched or straight, ligands, and preferably contain either a butyl or hexyl ligand. These ligands provide the preferential binding interaction which is exploited in the separation, purification and/or isolation methods of the present invention. One of ordinary skill in the art will appreciate that hydrophobic resins may include ligands in addition to or in place of these alkyl ligands, which will also be useful in the method of the present invention. Examples of such ligands are also described above. The following non-limiting examples illustrate the methods of the presently disclosed invention. The following general methods were or could be used.

Plasmids for gene therapy applications were extracted from a suitable host bacterium, for example *Escherchia coli,* following fermentation. In the following exemplification of the disclosed invention, *E*. *coli* STBL-2, which contains plasmid pEIA-K2 was used. Plasmid pEI-A-K2 is a pUC plasmid derivative that contains a suppresser gene from adenovirus Type 5, and contains a kanamycin gene as a selectable marker. Fermentation was conducted aerobically in a suitable yeast extract/glucose medium containing inorganic salts, such as potassium mono basic phosphate, sodium dibasic phosphate, ammonium sulfate and magnesium sulfate at a pH of from 6.5 to 7.8, preferably 7.0, and at a temperature of 37°C. Aeration was set to one volume of air per volume of medium and the agitation set to 800 rpm. Cells were grown in this mode until the glucose was exhausted from the medium, then the DO of the fermentor was controlled by glucose feed and agitation. The feed contained a concentrated solution of glucose (160 g/L) and yeast extract (80 g/L) and salts (1.5 g/L ammonium sulfate, MgSO₄, 1.5 g/L in phosphate buffer). After completion of fermentation, the cells were harvested by centrifugation or by filtration through ultra or microfiltration membranes, and washed with TE (see below) buffer, pH 7.4. Cells were lysed by contacting the suspension with an equal volume of a solution of 0.15N to 0.2N NaOH and 1% sodium dodecyl sulfate (pH 11.5 to 13) with gentle mixing. The alkaline solution was neutralized with a potassium acetate solution. The material was then clarified by either centrifugation or by passing the suspension through a series of depth filters. Plasmid solutions are concentrated by ultrafiltration membranes and diafilered against TE buffer, pH 7. The diafilter retentate can be applied directly to the media described herein.

The plasmid DNA content of the lysate is generally less than 2% of the total nucleic acid with the bulk of the contents being RNA or chromosomal DNA. In addition, the lysate is contaminated with endotoxin and cellular proteins.

The present invention also provides methods of small-, laboratory- or bench-scale production of isolated or purified DNA, and equipment columns and separators useful therein. One of ordinary skill in the art will appreciate that small-scale production of plasmid DNA introduces different challenges, as compared to large-scale production. Specifically, the small-scale separation entails separation of a larger proportion of contaminating RNA in the starting material, such that while the plasmid to RNA ratio in a large-scale starting material may be about 2% (wt/wt), as noted above, the small scale ratio is about 0.1% (wt/wt). Moreover, the culture volume of the small-scale samples are generally about 2 mL to about 2L, as opposed to about 5L to about 1000L in the large-scale separation. The small-scale separations of the present invention involve about 100 µg to about 1mg of plasmid with a reactor or column bed volume of about 1 to about 20 ml; usually in the range of about 10 ml to about 15 ml. The present invention provides therefore, efficient small-scale production of isolated and/or purified DNA, preferably supercoiled DNA, from impurities, such as endotoxin, RNA and relaxed DNA.

While various procedures are used in the present exemplification, one of ordinary skill will appreciate that other preparative methods and starting materials may be used in the presently disclosed invention.

### Example 1: Endotoxin removal using Butyl Hydrophobic Interaction Chromatography (Small scale)

*E. coli* cells harboring the plasmid pEIA-K2 were grown, and lysed using chemical methods, and clarified through filtration methods. All buffers used throughout were filtered through 0.2 µm filter, and samples for endotoxin were stored in polystyrene sample tubes.

A diafiltration retentate (∼ 400ml) was dialyzed into TE pH 7.4 (50ml Tris, 10mM EDTA adjusted to pH 7.4 with HCI) was used for the experiment. Ammonium sulfate (AS) as required to make the sample 2M was added to 100 ml of TE plus 2M AS, pH 7.4 buffer and partially dissolved. This solution was added to the dialyzed sample to make a final volume of 575 ml, of which 475 ml was used for the experiment.

A Butyl 650S column (Butyl 650S resin from TosoHaas Inc., 156 Keystone Drive, Montgomeryville, PA 18936) of 2.6 cm diameter and 15 cm bed height, of approximately 75 ml bed volume was packed and equilibrated with TE buffer, pH 7.4, containing 2M AS. The sample was loaded at a flow rate of 5 ml/min. The flow through was collected, and samples were taken for analysis (DNA concentration, agarose gel, and endotoxin assay. Endotoxin assay was performed with spikes and samples were diluted appropriately to obtain PPC (Positive Product Control) recoveries in the range considered acceptable. Endotoxin concentrations were determined using the BioWhittaker Kinetic-QCL Chromogenic LAL assay as described in BW publication No. P50-650U-5, Kinetic-QCL Test Kit Manual. Following the sample load, TE containing 2M ammonium sulfate was flowed through the column, and collected and sampled. The column was subsequently washed with TE buffer - pH 7.4, USP purified water, and cleaned with 0.5N sodium hydroxide, and rinsed with >15 volumes of USP purified water. Endotoxin was present in each of these washes as shown below in Table 1. In addition to this outstanding endotoxin removal efficiency, significant amount of RNA, protein, and DNA fragments were removed, leaving the sample significantly purified.

**Table 1**

| **Sample** | **DNA conc. (mg/ml)** | **Endotoxin EU/ml** | **Total EU units** | **% Endotoxin** | **EU per Mg of DNA** |
|---|---|---|---|---|---|
| Load | 0.70 | 472,200 | 22,400,000 | 100 | 674,571 |
| Flow through | 0.38 | 1.64 | 771 | 0.003 | 4.31 |
| Wash | 0.56 | 7.48 | 1,196 | 0.005 | 13.42 |
| Endotoxin capacity per ml of resin: | | | | 3 million | |
| EU/ml | | | | | |
| Endotoxin reduction in sample: | | | | 99.992% | |

### Example 2: Endotoxin removal using Butyl Hydrophobic Interaction Chromatography (large scale)

*E. coli* cells harboring the plasmid pEIA-K2 was grown, and lysed using chemical methods, and clarified through filtration methods.

The diafiltration retentate (- 650ml) was dialyzed into TE pH 7.4 (50mM Tris, 10mM EDTA adjusted to pH 7.4 with HCl) and ammonium sulfate required to make the sample 2M was added to 1200 ml of TE containing 2M AS, pH 7.4 buffer and dissolved. The volume of this solution was made up to 1300 ml. This solution was added to the dialyzed sample to make a final volume of 1950 ml, and pH was adjusted to 7.4 using HCI.

A Butyl 650S column of 5 cm diameter and 15 cm bed height, of approximately 275 ml bed volume was packed and equilibrated with TE buffer, pH 7.4, containing 2M AS. The sample was loaded at a flow rate of 20 ml/min. The flow through was collected, and samples were taken for analysis (DNA concentration, agarose gel, and endotoxin assay, as described above). Following the sample load, TE containing 2M ammonium sulfate was flowed through the column, and collected and sampled. The column was subsequently washed with TE buffer - pH 7.4, USP purified water, and cleaned with 0.5N sodium hydroxide, and rinsed with >15 volumes of USP purified water. Endotoxin was present in each of these washes as shown below in Table 2. In addition to this extremely outstanding endotoxin removal efficiency, significant amount of RNA, protein, and DNA fragments were removed, leaving the sample significantly purified.

**Table 2**

| **Sample** | **DNA conc. (mg/ml)** | **Endotoxin EU/ml** | **Total EU** | **% Endotoxin** | **EU per mg of DNA** |
|---|---|---|---|---|---|
| Load | 1.59 | 271500 | 176,475, 000 | 100 | 170,754 |
| Flowthrough + Wash | 0.34 | <0.5 | 1325 | 0.007 | 1.45 |
| Endotoxin capacity per ml of resin: | | | | 0.64 | |
| million EU/ml | | | | | |
| Endotoxin reduction in sample: | | | | 99.993% | |

### Example 3: Endotoxin removal using Hexyl Hydrophobic Interaction Chromatography (Small scale)

*E. coli* cells harboring the plasmid pEIA-K2 was grown, and lysed using chemical methods, and clarified through centrifugation methods. The supernatant was dialyzed into a 20mM potassium phosphate buffer (20mM potassium phosphate monobasic solution combined with 20mM potassium phosphate dibasic in a proportion to obtain a pH of 6.8), pH 6.8 and ammonium sulfate required to make the sample 2M was added to 20 ml of KPB (20mM potassium phosphate buffer) containing 2M AS, pH 6.8 buffer and dissolved. This solution was added to 5 ml of dialyzed sample to make a final volume of 25 ml and pH was adjusted to 6.8.

A Hexyl 650C (TosoHaas) column of 1.6 cm diameter and 4 cm bed height, of approximately 8 ml bed volume was packed and equilibrated with KPB, pH 6.8, containing 2M AS. The sample was loaded at a flow rate of 2 ml/min. The flow through was collected, and samples were taken for analysis (DNA concentration, agarose gel, and endotoxin assay, see above). Following the sample load, KPB containing 2M ammonium sulfate was allowed to flow through the column, collected and sampled. The column was subsequently washed with KPB pH 6.8, USP purified water, and cleaned with 0.5N sodium hydroxide, and rinsed with >15 volumes of USP purified water. Endotoxin was present in each of these washes as shown below in Table 3. In addition to this extremely outstanding endotoxin removal efficiency, significant amount of RNA, protein, and DNA fragments were removed, leaving the sample significantly purified.

**Table 3**

| Sample | DNA conc. (mg/ml) | Endotoxin EU/ml | Total EU units | % Endotoxin | EU per Mg of DNA |
|---|---|---|---|---|---|
| Load | 2.43 | 593500 | 29,675,000 | 100 | 244,238 |
| Flow through + Wash | 0.037 | 0.5 | 35 | 0.0001 | 14 |
| Endotoxin capacity per ml of resin: | | | | 3.7 million | |
| EU/ml | | | | | |
| Endotoxin reduction in sample: | | | | 99.999% | |

### Example 4: Endotoxin removal using Octyl Hydrophobic Interaction Chromatography (Small scale)

*E. coli* cells harboring the plasmid pEIA-K2 was grown, and lysed using chemical methods, and clarified through centrifugation methods as described above. The supernatant was used for the experiment. Ammonium sulfate required to make the sample 2M was added to 20 ml of TE plus 2M AS, pH 7.4 buffer and dissolved. This solution was added to 10 ml of dialyzed sample to make a final volume of 25 ml and pH was adjusted to 7.4.

An Octyl Sepharose 4 Fast Flow column (Amersham Pharmacia Biotech, Piscataway, NJ) of 1.0 cm diameter and 10 cm bed height, of approximately 8 ml bed volume was packed and equilibrated with TE, pH 7.4, containing 2M AS. The sample was loaded at a flow rate of 2 ml/min. The flow through was collected, and samples were taken for analysis (DNA concentration, agarose gel, and endotoxin assay). Following the sample load, TE containing 2M ammonium sulfate was flowed through the column, collected and sampled. The column was subsequently washed with TE pH 7.4, USP purified water, and cleaned with 0.5N sodium hydroxide, and rinsed with >15 volumes of USP purified water. Endotoxin was present in each of these washes as shown below in Table 4.

**Table 4**

| **Sample** | **DNA conc. (mg/ml)** | **Endotoxin EU/ml** | **Total EU units** | **% Endotoxin** | **EU per mg of DNA** |
|---|---|---|---|---|---|
| Load | 2.43 | 593500 | 59,350,000 | 100 | 244,238 |
| Flow through + Wash | 0.037 | 32 | 2240 | 0.004 | 280 |
| Endotoxin capacity per ml of resin: | | | | 7.41 | |
| million EU/ml | | | | | |
| Endotoxin reduction in sample: | | | | 99.996% | |

### Example 5: Separation of the supercoiled and relaxed forms of the plasmid DNA using Butyl Hydrophobic Interaction Chromatography-Gradient Elution

*E. coli* cells harboring the plasmid pEIA-K2 were grown, and lysed using chemical methods, and clarified through filtration methods, as described above. Gross purification of the plasmid DNA to eliminate major contaminants such as endotoxin, RNA, protein, chromosomal DNA, etc. was performed using butyl hydrophobic interaction chromatography, where, at a concentration of 2M Ammonium sulfate, the plasmid DNA flows through the column while the contaminants bind to the column (see above).

The flow through containing the plasmid DNA was dialyzed and processed on an anion exchange (Bio Sepra) column that did not provide any additional purification. Processing through the Q anion exchange column and diafiltrations are not necessary to achieve the separation on the butyl column. The elution from the Q anion exchange column was diafiltered using a 30kD regenerated cellulose membrane (0.1m² , Millipore Corporation). The dialyzed material was adjusted to 3M ammonium sulfate using solid ammonium sulfate.

A Butyl (Toyopearl Butyl 650S - TosoHaas) column of 2.6 cm diameter and approximately 15 cm height was packed at a flow rate of 15-20mUmin. The column was equilibrated with 3M ammonium sulfate in Tris - EDTA buffer pH 7.4. The sample was loaded at a flow rate of 5 ml/min. The plasmid was bound to the column at 3M ammonium sulfate. The column was then washed with 2-3 column volumes of a 3M ammonium sulfate solution in buffer. The column was then eluted with a gradient of ammonium sulfate concentration from 3M to 1M over 6 bed volumes. During the gradient elution, two peaks resulted, the first peak containing the relaxed form of the plasmid DNA, and the second peak containing the supercoiled form of plasmid DNA as evidenced in agarose gels of fractions (Figure 4A). The chromatogram is shown in Figure 4B. The results were further confirmed by an HPLC assay used to determine the percentage of the two forms (Table 5).

Within the limits of sensitivity of the assay, it was confirmed that the second peak contained 85% supercoiled form, whereas the starting material only contained 50-60% supercoiled form. At least 90% of the starting supercoiled plasmid DNA was recovered. The resolution of the peaks were adequate to effect the separation, even with 15 cm column height. This demonstrates the effective separation of supercoiled and relaxed forms of the plasmid using Butyl hydrophobic interaction chromatography. In addition to this excellent separation, residual amount of RNA, protein, and endotoxin could be removed resulting in product that meets the specifications for gene therapy.

**Table 5**

| **Sample** | **% Supercoiled** |
|---|---|
| Starting material | 63 |
| Peak 1 Fraction | 8 |
| Peak 2 Fraction | 83 |

### Example 6: Separation of the supercoiled and relaxed forms of the plasmid DNA using Butyl Hydrophobic Interaction Chromatography-Gradient Elution Long column

*E. coli* cells harboring the plasmid pEIA-K2 were grown, and lysed using chemical methods, and clarified through filtration methods, as described above. Gross purification of the plasmid DNA to eliminate major contaminants such as endotoxin, RNA, protein, chromosomal DNA, etc. was performed using butyl hydrophobic interaction chromatography, where, at a concentration of 2M Ammonium sulfate, the plasmid DNA flows through the column while the contaminants bind to the column (see above). The flow through containing the plasmid DNA was dialyzed and processed on an anion exchange column that did not provide any additional purification. The elution from the Q column was diafiltered using a 30kD regenerated cellulose membrane. The dialyzed material was adjusted to 3M ammonium sulfate using solid ammonium sulfate, as described above in Example 5.

A Butyl (Toyopearl Butyl 650S - TosoHaas) column of 2.6 cm diameter and approximately 30 cm height was packed at a flow rate of 15-20ml/min. The column was equilibrated with 3M ammonium sulfate in Tris - EDTA buffer pH 7.4. The sample was loaded at a flow rate of 5 ml/min. The plasmid was bound to the column at 3M ammonium sulfate. The column was then washed with 2-3 bed volumes with a 3M ammonium sulfate buffer solution. The column was then eluted with a gradient of ammonium sulfate concentration from 3M to 1M over 6 bed volumes. During the gradient elution, two peaks resulted, the first peak containing the relaxed form of the plasmid DNA, and the second peak containing the supercoiled form of plasmid DNA as evidenced in agarose gels of fractions (Figure 5A). The chromatogram is shown in Figure 5B. The results were further confirmed by an HPLC assay used to determine the percentage of the two forms (Table 6).

Within the limits of sensitivity of the assay, it was confirmed that the second peak contained 90% supercoiled form, whereas the starting material only contained 50% supercoiled form. Baseline resolution of the peaks was obtained with the longer column. This example clearly demonstrates the effective separation of supercoiled and relaxed forms of the plasmid using butyl hydrophobic interaction chromatography. In addition to this excellent separation, residual amounts of RNA, protein, and endotoxin could be removed resulting in product that meets the specifications for gene therapy.

**Table 6**

| **Sample** | **% Supercoiled** |
|---|---|
| Starting material | 53 |
| Peak 1 Fraction 36 | 15 |
| Peak 2 Fraction 47 | 83 |
| Peak 2 Fraction 49 | 95 |
| Peak 2 Fraction 52 | 91 |

### Example 7: Separation of the supercoiled and relaxed forms of the plasmid DNA using Butyl Hydrophobic Interaction Chromatography - Step Elution Long column

*E. coli* cells harboring the plasmid pEIA-K2 were grown, and lysed using chemical methods, and clarified through filtration methods, as described above. Gross purification of the plasmid DNA to eliminate major contaminants such as endotoxin, RNA, protein, chromosomal DNA, etc. was performed using butyl hydrophobic interaction chromatography, where, at a concentration of 2M Ammonium sulfate, the plasmid DNA flows through the column while the contaminants bind to the column (see Example 1 above). The flow through and wash were pooled and concentrated using a 30kD ultrafiltration membrane using tangential flow filtration. The concentrated plasmid DNA was adjusted to 3M ammonium sulfate using solid ammonium sulfate.

A Butyl (Toyopearl Butyl 650S - TosoHaas) column of 1 cm diameter and approximately 30 cm height was packed at a flow rate of 6 ml/min. The column was equilibrated with 3M ammonium sulfate in Tris - EDTA buffer pH 7.4. The sample was loaded at a flow rate of 2 ml/min. The plasmid was bound to the column at 3M ammonium sulfate. The column was then washed with 2-3 bed volumes with a 3M ammonium sulfate buffer solution. The column was then eluted with various concentrations of ammonium sulfate - 2.8M, 2.7M, 2.6M, 2.55M, 2.5M, 2.4M, 1M - using 2-3 column volumes. Peaks were observed in the 2.4M and 1M elutions. Agarose gel electrophoresis of the peaks is shown in Figure 6A. The gel indicates clear separation of the supercoiled and relaxed forms. The 2.4M elution contains the relaxed form, while the 1M elution contains the supercoiled DNA. The chromatogram is shown in Figure 6B. The results were further confirmed by an HPLC assay used to determine the percentage of the two forms (Table 7).

Within the limits of sensitivity of the assay, it was confirmed that the second peak contained 93% supercoiled form, whereas the starting material only contained 62% supercoiled form. These results were confirmed by subsequent experiments where 2.3M and 2.2M elutions did not provide the resolution, and the 2.4M elution repeatedly provided significant removal of the relaxed form, thereby enriching for the supercoiled form in the 1M elution. The separation accomplished using step elution is significant in large scale separations, which are more reliably performed using step elutions. This example clearly demonstrates the effective separation of supercoiled and relaxed forms of the plasmid using step elution of butyl hydrophobic interaction chromatography. In addition to this excellent separation, residual amount of RNA, protein, and endotoxin could be removed resulting in product that meets the specifications for gene therapy.

**Table 7**

| **Sample** | **% Supercoiled** |
|---|---|
| Starting material | 62 |
| 2.4M Elution | 8 |
| 1M Elution | 93 |

### Example 8: Separation of the supercoiled and relaxed forms of the plasmid DNA using Hexyl Hydrophobic Interaction Chromatography-Gradient Elution Long column

*E. coli* cells harboring the plasmid pEIA-K2 were grown, and lysed using chemical methods, and clarified through filtration methods, as described above. Gross purification of the plasmid DNA to eliminate major contaminants such as endotoxin, RNA, protein, chromosomal DNA, etc. was performed using butyl hydrophobic interaction chromatography, where, at a concentration of 2M Ammonium sulfate, the plasmid DNA flows through the column while the contaminants bind to the column (see, for example, Example 1 above). The flow through containing the plasmid DNA was dialyzed and processed on an anion exchange column that did not provide any additional purification. The elution from the Q column was diafiltered using a 30kD regenerated cellulose membrane. The dialyzed material was adjusted to 3M ammonium sulfate using solid ammonium sulfate (see above).

A Hexyl (Toyopearl Hexyl 650C - TosoHaas) column of 1 cm diameter and approximately 30 cm height was packed at a flow rate of 5 ml/min. The column was equilibrated with 3M ammonium sulfate in Tris-EDTA buffer pH 7.4. The sample was loaded at a flow rate of 2 ml/min. The plasmid was bound to the column at 3M ammonium sulfate. The column was then washed with 2-3 bed volumes with a 3M ammonium sulfate buffer solution. The column was then eluted with a gradient of ammonium sulfate concentration from 3M to 1M over 6 bed volumes. During the gradient elution, two peaks resulted, the first peak containing predominantly the relaxed form of the plasmid DNA, and the second peak containing predominantly the supercoiled form of plasmid DNA as evidenced in agarose gels of fractions (Figure 7A).

The chromatogram is shown in Figure 7B. Qualitatively, the second peak contained significantly higher proportion of supercoiled plasmid than the starting material based on agarose gel electrophoresis. Excellent resolution of the peaks were obtained, considering the fact that the bead size was 100 :m for the Hexyl, compared to 35 :m for the Butyl.

### Example 9: Endotoxin removal using Butyl Hydrophobic Interaction Chromatography (using sodium chloride)

*E. coli* cells harboring the plasmid pEIA-K2 was grown, and lysed using chemical methods, and clarified through centrifugation methods. The supernatant was used for the experiment. The sample was purified through an anion exchange column (Q Hyper D - BIOSEPRA Inc.). A 2M sodium chloride elution from the column was used for this experiment. The sample was present in 50mM Tris 10mM EDTA pH 7.4 buffer with 2M NaCl. A Butyl HIC column (using Butyl 650S resin - TosoHaas) of diameter 1 cm and height 20 cm of approximately 10ml volume was packed and equilibrated with TE containing 2M sodium chloride. The sample was loaded at a flow rate of 2 ml/min. The flow through was collected, and samples were taken for analysis (DNA concentration, agarose gel, and endotoxin assay). Following the sample load, TE containing 2M ammonium sulfate was flowed through the column, collected and sampled. The column was subsequently washed with TE pH 7.4.

**Table 8**

| **Sample** | **DNA conc. (mg/ml)** | **Endotoxin EU/ml** | **Total Eunits EU** | **% Endotoxin** | **EU per mg of DNA** |
|---|---|---|---|---|---|
| Load | 0.27 | 642 | 16,050 | 100 | 2377 |
| Wash | 0.13 | 5 | 350 | 2 | 38 |
| Endotoxin capacity per ml of resin: | | | **1570 EU/ml** | | |
| Endotoxin reduction in sample: | | | **98 %** | | |

### Example 10: Small Scale Plasmid Purification with Butyl Hydrophobic Interaction Chromatography

Small-scale plasmid DNA purification is performed using commercially available kits, the most commonly known of which is a Qiagen's Miniprep kit. The methods described herein may be used for purification of plasmid DNA to provide several advantages over commercial kits. The following example demonstrates the use of the hydrophobic interaction chromatography method of the present invention for purification of plasmid DNA on a small scale.

The starting material for purification was obtained through standard methods. Specifically, E. coli cells harboring plasmid of approximately 4.65 Kb size was grown in Luria Broth containing 100µg/ml of ampicillin at 37°C. The cells were harvested at an OD of 2.7. The cells were removed from the media through centrifugation. Subsequently, the cell pellet was resuspended in 50mM Tris-HCl, 10mM EDTA pH8.0. An equal volume of 200mM NaOH, 1% SDS solution was added, mixed well and incubated at room temperature for 5 minutes. This step results in lysis of the cells, releasing the cell contents, including plasmid DNA. Neutralization solution consisting of 3.1M potassium acetate (pH 5.5) was added in equal (original) volume and mixed well. The neutralized lysate was then filtered through cheese cloth and filters to remove the precipitate. The clarified lysate was precipitated with 70% isopropanol (adding 2.1ml isopropanol per 3 ml of clarified lysate). The precipitate was separated through centrifugation and the pellet was washed with 70% ethanol, dried and dissolved in 10mM Tris-HCl, 0.1mm EDTA buffer, pH 8.0. This preparation was frozen at -20°C until use and was the starting material for the purification experiments.

A Butyl 650S column with a bed volume of 20 ml and bed height of 10 cm was packed in a 1.6cm diameter column (Pharmacia XK16/20) and equilibrated with 2.2M ammonium sulfate (AS) in 50mM Tris-HCl, 10mM EDTA (TE) buffer, pH 7.4. The sample for load was prepared by adding solid ammonium sulfate to a final concentration of 2.2M. The sample was diluted with 2.2M AS in Tris - EDTA, pH 7.4 to 10 ml.

Purification conditions were designed to allow the plasmid DNA to be collected in the flow through and the contaminants were bound to the resin.

The sample was loaded at 2 ml/min and flow through was collected. The column was washed with 35 ml of 2.2M AS in TE buffer and collected as Wash 1(10 ml), Wash 2 (14.5 ml), and Wash 3 fractions (10ml). A peak resulted during the wash. The column was eluted with 55 ml of 1M AS in TE buffer, pH 7.4 and collected as 1M Wash 1 (10ml) and 1M Wash 2 (45ml). A peak resulted during 1M AS elution. The column was eluted with 50 ml of USP - Purified Water. A peak resulted. The table below shows the total nucleic acid present in each of the fractions above. The concentrations were calculated based on the absorbance at 260nm (Conc. (µg/ml) = A260 * 50)

**Table 9**

| **Column fractions** | **Conc.** | **Mass** |
|---|---|---|
| | **(µg/ml)** | **(µg)** |
| Load | 550.0 | 5503 |
| Wash 1 | 7.6 | 76 |
| Wash 2 | 40.4 | 607 |
| Wash 3 | 5.0 | 50 |
| **Wash pool (Plasmid DNA)** | **20.9** | **733** |
| 1M elution | 35.8 | 1611 |
| Water elution | 30.5 | 1523 |

An agarose gel electrophoresis of the samples were preformed. Figure 8 shows a photocopy of the gel. The photograph of the gel shows the plasmid DNA in the Wash fractions (Lane 3, 4, 5). In comparison with the load sample (Lane 2) no visible RNA is seen in the Wash fractions. The elution fractions contain RNA as seen in Lanes 6 and 7. The endotoxin in the Wash pool was measured using a Kinetic QCL endotoxin assay. No endotoxin was detected in the sample at the sensitivity level of the assay, which was .005 EU/ml. The yield of plasmid was 733 µg from 100 ml of the culture which is similar to that obtainable with commercial kits.

Production of pharmaceutical-grade plasmid DNA, Magda Marquet, Nancy Horn, Jennifer Meek, Gregg Budahazi, U.S. Patent Number. 5,561,064

Concentration and size-fractionation of nucleic acids and viruses in porous media, Cole, Kenneth D., U.S. Patent Number: 5,707,850

Purification of plasmid DNA during column chromatography, Nancy Horn, Greg Budahazi, Magda Marquet, U.S. Patent Number: 5,707,812

## Claims

1. A method for purifying plasmid DNA from a mixture of same containing at least one host cell impurity comprising the following steps:
(a) forming a solution by adding sufficient salt to said mixture to allow selective binding of said at least one host cell impurity to a hydrophobic interaction media, wherein said salt is ammonium sulfate or sodium salts in a concentration range of 2M to 4M;
(b) contacting said solution containing plasmid DNA with said hydrophobic interaction media under conditions that said at least one impurity binds to the hydrophobic interaction media to form a complex; and
(c) collecting unbound plasmid DNA from said complex as flow-through from the loading process;
wherein said method is conducted in the absence of organic solvents, detergents, glycols, hexamine cobalt, spermidine, and polyvinylpyrrolidone.

2. The method of claim 1 wherein the at least one impurity is selected from the group consisting of RNA, endotoxin, chromosomal DNA and protein.

3. The method for claim 1 wherein the at least one impurity is an endotoxin.

4. The method of claim 1 wherein ammonium sulfate is present at a concentration of 2M.

5. The method of claim 1 wherein the sodium salt is sodium chloride.

6. The method of claim 5 wherein the sodium salt is sodium chloride in a concentration of 2M.

7. The method of claim 1 wherein the solution has a pH in the range of 6.8 to 7.4.

8. The method of claim 1 wherein the solution has a pH of 7.4.

9. A method of separating supercoiled plasmid DNA from a mixture of supercoiled plasmid DNA and relaxed plasmid DNA and, optionally, at least one host cell impurity comprising the following steps:
(a) forming a solution by adding a salt to the mixture of supercoiled DNA and relaxed plasmid DNA and, when present, said at least one host cell impurity;
(b) contacting the solution with a hydrophobic interaction media under a first condition where both the supercoiled plasmid DNA and relaxed plasmid DNA bind to the hydrophobic interaction media to form a bound first mixture;
(c) altering the first condition surrounding the bound first mixture to a second condition to remove relaxed plasmid DNA from the bound first mixture to form separate components containing a second bound mixture and relaxed plasmid DNA; and
(d) modifying the second condition surrounding the said second bound mixture to a third condition to remove supercoiled plasmid DNA from said second bound mixture to form separate components containing hydrophobic interaction media and supercoiled plasmid DNA.
wherein said method is conducted in the absence of organic solvents, detergents, glycols, hexamine cobalt, spermidine, and polyvinylpyrrolidone.

10. The method of claim 9 wherein the at least one host cell impurity is selected from the group consisting of RNA, endotoxin, chromosomal DNA and protein.

11. The method for claim 9 wherein the at least one host cell impurity is an endotoxin.

12. The method of claim 9 wherein the salt comprises an anion or cation selected from the group consisting of SO₄²⁻, Cl⁻, Br⁻, NO₃⁻, Mg²⁺, Li⁺, Na⁺, K⁺, and NH₄⁺.

13. The method of claim 12 wherein the salt is ammonium sulfate in a concentration range of 2.5M to 4M.

14. The method of claim 9 wherein the first condition comprises equilibrating said media with a salt solution containing ammonium sulfate which is present in a concentration range of 2.5M to 4M.

15. The method of claim 9 wherein the second condition comprises washing the media with a salt solution containing ammonium sulfate in a concentration of 2.35M to 2.45M.

16. The method of claim 9 wherein the said third condition comprises washing said second bound mixture with a salt solution containing ammonium sulfate in a concentration of 1 M to 2.3 M.

17. A method of separating endotoxin from plasmid DNA comprising contacting a mixture of endotoxin and plasmid DNA with a hydrophobic interaction media under conditions where said endotoxin binds said hydrophobic interaction media to form a complex and separating said plasmid DNA and said complex by collecting said plasmid DNA as flow-through from the loading process,
wherein said method is conducted in the absence of organic solvents, detergents, glycols, hexamine cobalt, spermidine, and polyvinylpyrrolidone.

18. The method of claim 17, wherein said mixture further comprises an ammonium salt in a concentration range of 1.5 to 4M.

19. The method of claim 18, wherein said ammonium salt is ammonium sulfate which is present at a concentration of 2M.

20. The method of claim 17, wherein said mixture has a pH in the range of 6.8 to 7.4.

21. The method of claim 20, wherein the pH is 7.4.

22. A method of separating supercoiled plasmid DNA from relaxed plasmid DNA comprising contacting a mixture of supercoiled plasmid DNA and relaxed plasmid DNA with a hydrophobic interaction media under a first condition where both the supercoiled plasmid DNA and the relaxed plasmid DNA bind to said hydrophobic interaction media to form a bound first mixture, altering said first condition surrounding the bound first mixture to a second condition to remove said relaxed plasmid DNA from said bound first mixture to form separate components containing a second bound mixture and said relaxed plasmid DNA, and modifying the second condition surrounding said second bound mixture to a third condition to remove said supercoiled plasmid DNA from said second bound mixture to form separate components containing said hydrophobic interaction media and said supercoiled plasmid DNA;
wherein said method is conducted in the absence of organic solvents, detergents, glycols, hexamine cobalt, spermidine, and polyvinylpyrrolidone.

23. The method of claim 22 wherein said first condition comprises equilibrating said media with a salt solution containing ammonium sulfate in a concentration range of 2.5M to 4M.

24. The method of claim 23 wherein said second condition comprises washing said bound first mixture with a salt solution containing ammonium sulfate in a concentration of 2.35M to 2.45M.

25. The method of claim 24 wherein said third condition comprises washing said second bound mixture with a salt solution containing ammonium sulfate in a concentration of 1 M to 2.3M.

26. The method of any one of claims 9 and 22, wherein said altering and said modifying are combined in a continuous process comprising gradient elution of said relaxed plasmid DNA and supercoiled plasmid DNA by mixing said bound first mixture with an ammonium sulfate containing salt solution with a continuously varying concentration of ammonium sulfate, said concentration varying from 3M to 1 M ammonium sulfate, and said relaxed plasmid DNA is collected in a first eluted volume and said supercoiled plasmid DNA is collected in a second eluted volume.

27. The method of claim 22 wherein said separate relaxed plasmid DNA component and said separate supercoiled plasmid DNA are collected and isolated.

28. A method for enriching the amount of supercoiled plasmid DNA relative to relaxed plasmid DNA in a mixture thereof, the method comprising:
(1) interacting the mixture containing supercoiled plasmid DNA and relaxed plasmid DNA with a hydrophobic interactive media comprising an alkyl moiety under ionic conditions wherein the supercoiled plasmid DNA preferentially binds to the hydrophobic interactive media;
(2) treating the hydrophobic interactive media containing the relaxed and supercoiled plasmid DNA under ionic conditions that allow the preferential removal of the relaxed plasmid DNA; and
(3) eluting the supercoiled plasmid DNA from the hydrophobic interactive media;
wherein said method is conducted in the absence of organic solvents, detergents, glycols, hexamine cobalt, sperdimine, and polyvinylpyrrolidone.

29. A method for removing lipopolysaccharide (LPS) from a composition containing plasmid DNA, the method comprising:
(1) interacting the mixture comprising the plasmid DNA and LPS with a hydrophobic interaction media comprising an alkyl moiety, wherein the interacting is under ionic conditions where the LPS preferentially binds to the hydrophobic interactive media relative to the plasmid DNA; and
(2) treating the hydrophobic interactive media containing the plasmid DNA and LPS with ionic conditions that allow the selective removal of the DNA,
wherein said method is conducted in the absence of organic solvents, detergents, glycols, hexamine cobalt, spermidine, and polyvinylpyrrolidone.

30. The method of any one of claims 1, 9, 17 or 22 wherein the hydrophobic interaction media comprises a chromatography support with pendent hydrophobic groups.

31. The method of claim 30 wherein said pendent groups are selected from the group consisting of C₃ to C₁₀ alkyl groups.

32. The method of claim 30 in respect of claims 9 or 17 wherein the hydrophobic interaction media is selected from the group consisting of a methacrylate polymer or copolymer backbone bound to at least one of a propyl, butyl, hexyl, octyl, nonyl, or a mixture of these as pendent hydrophobic groups.

33. The method of claim 30 in respect of claim 1 wherein the hydrophobic interaction media is selected from the group consisting of a methacrylate polymer or copolymer backbone bound to at least one of a propyl, butyl, hexyl, octyl, nonyl or decyl hydrophobic groups.

34. The method of claim 30 in respect of claim 22 wherein the hydrophobic interaction media is a methacrylate polymer or copolymer backbone bound to at least one of a propyl, butyl, pentyl, hexyl, heptyl, octyl, nonyl or decyl ligand.

35. The method of claim 30 wherein the media is at least one of a methacrylate ethylene glycol copolymer backbone or a cross-linked agarose.

36. A method of claim 30 wherein the media is a resin in the form of beads in the size range of 15 to 100 µm.

37. The method of claim 35, wherein the salt is ammonium sulfate in a concentration range of 2.5M to 4M.

## Patentansprüche

1. Verfahren zur Reinigung von Plasmid-DNA aus einem Gemisch derselben, enthaltend zumindest eine Wirtszellenverunreinigung, umfassend die folgenden Schritte:
(a) Bilden einer Lösung durch Hinzufügen von ausreichend Salz zu dem Gemisch, um eine selektive Bindung der zumindest einen Wirtszellenverunreinigung an ein hydrophobes Wechselwirkungsmedium zu ermöglichen, wobei das Salz Ammoniumsulfat oder Natriumsalz in einem Konzentrationsbereich von 2 M bis 4 M ist;
(b) Kontaktieren der Lösung, enthaltend Plasmid-DNA, mit dem hydrophoben Wechselwirkungsmedium unter Bedingungen, unter denen die zumindest eine Verunreinigung an das hydrophobe Wechselwirkungsmedium bindet, um einen Komplex zu bilden; und
(c) Sammeln ungebundener Plasmid-DNA aus dem Komplex als Durchfluss von dem Beladungsprozess;
wobei das Verfahren in Abwesenheit von organischen Lösungsmitteln, Detergenzien, Glycolen, Hexaminkobalt, Spermidin und Polyvinylpyrrolidon durchgeführt wird.

2. Verfahren nach Anspruch 1, worin die zumindest eine Verunreinigung aus der aus RNA, Endotoxin, chromosomaler DNA und Protein bestehenden Gruppe ausgewählt ist.

3. Verfahren nach Anspruch 1, worin die zumindest eine Verunreinigung ein Endotoxin ist.

4. Verfahren nach Anspruch 1, worin Ammoniumsulfat in einer Konzentration von 2 M vorliegt.

5. Verfahren nach Anspruch 1, worin das Natriumsalz Natriumchlorid ist.

6. Verfahren nach Anspruch 1, worin das Natriumsalz Natriumchlorid in einer Konzentration von 2 M ist.

7. Verfahren nach Anspruch 1, worin die Lösung einen pH im Bereich von 6,8 bis 7,4 aufweist.

8. Verfahren nach Anspruch 1, worin die Lösung einen pH von 7,4 aufweist.

9. Verfahren zur Trennung von supergeknäulter Plasmid-DNA aus einem Gemisch von supergeknäulter Plasmid-DNA und entspannter Plasmid-DNA und gegebenenfalls zumindest einer Wirtszellenverunreinigung, umfassend die folgenden Schritte:
(a) Bilden einer Lösung durch Hinzufügen eines Salzes zu dem Gemisch von supergeknäulter Plasmid-DNA und entspannter Plasmid-DNA und, wo vorhanden, der zumindest einer Wirtszellenverunreinigung;
(b) Kontaktieren der Lösung mit einem hydrophoben Wechselwirkungsmedium unter einer ersten Bedingung, unter der sowohl die supergeknäulte Plasmid-DNA als auch die entspannte Plasmid-DNA an das hydrophobe Wechselwirkungsmedium binden, um ein gebundenes erstes Gemisch zu bilden;
(c) Verändern der ersten Bedingung, die das gebundene erste Gemisch umgibt, zu einer zweiten Bedingung, um entspannte Plasmid-DNA aus dem gebundenen ersten Gemisch zu entfernen, um separate Komponenten zu bilden, die ein zweites gebundenes Gemisch und entspannte Plasmid-DNA enthalten; und
(d) Modifizieren der zweiten Bedingung, die das zweite gebundene Gemisch umgibt, zu einer dritten Bedingung, um supergeknäulte Plasmid-DNA aus dem zweiten gebundenen Gemisch zu entfernen, um separate Komponenten zu bilden, die hydrophobes Wechselwirkungsmedium und supergeknäulte Plasmid-DNA enthalten,
wobei das Verfahren in Abwesenheit von organischen Lösungsmitteln, Detergenzien, Glycolen, Hexaminkobalt, Spermidin und Polyvinylpyrrolidon durchgeführt wird.

10. Verfahren nach Anspruch 9, worin die zumindest eine Wirtszellenverunreinigung aus der aus RNA, Endotoxin, chromosomaler DNA und Protein bestehenden Gruppe ausgewählt ist.

11. Verfahren nach Anspruch 9, worin die zumindest eine Wirtszellenverunreinigung ein Endotoxin ist.

12. Verfahren nach Anspruch 9, worin das Salz ein Anion oder Kation umfasst, das aus der aus SO₄²⁻, Cl⁻, Br⁻, NO₃⁻, Mg²⁺, Li⁺, Na⁺, K⁺ und NH₄⁺ bestehenden Gruppe ausgewählt ist.

13. Verfahren nach Anspruch 12, worin das Salz Ammoniumsulfat in einem Konzentrationsbereich von 2,5 M bis 4 M ist.

14. Verfahren nach Anspruch 9, worin die erste Bedingung Äquilibrieren des Mediums mit einer Salzlösung umfasst, die Ammoniumsulfat enthält, das in einem Konzentrationsbereich von 2,5 M bis 4 M vorliegt.

15. Verfahren nach Anspruch 9, worin die zweite Bedingung Waschen des Mediums mit einer Salzlösung umfasst, die Ammoniumsulfat in einer Konzentration von 2,35 M bis 2,45 M enthält.

16. Verfahren nach Anspruch 9, worin die dritte Bedingung Waschen des zweiten gebundenen Gemischs mit einer Salzlösung umfasst, die Ammoniumsulfat in einer Konzentration von 1 M bis 2,3 M enthält.

17. Verfahren zur Trennung von Endotoxin von Plasmid-DNA, umfassend Kontaktieren eines Gemischs aus Endotoxin und Plasmid-DNA mit hydrophobem Wechselwirkungsmedium unter Bedingungen, bei denen das Endotoxin das hydrophobe Wechselwirkungsmaterial bindet, um einen Komplex zu bilden, sowie das Trennen der Plasmid-DNA und des Komplexes durch Sammeln der Plasmid-DNA als Durchfluss von dem Beladungsprozess,
wobei das Verfahren in Abwesenheit von organischen Lösungsmitteln, Detergenzien, Glycolen, Hexaminkobalt, Spermidin und Polyvinylpyrrolidon durchgeführt wird.

18. Verfahren nach Anspruch 17, worin das Gemisch weiters ein ein Ammoniumsalz in einem Konzentrationsbereich von 1,5 bis 4 M umfasst.

19. Verfahren nach Anspruch 18, worin das Ammoniumsalz Ammoniumsulfat ist, das in einer Konzentration von 2 M vorliegt.

20. Verfahren nach Anspruch 17, worin das Gemisch einen pH im Bereich von 6,8 bis 7,4 aufweist.

21. Verfahren nach Anspruch 20, worin der pH bei 7,4 liegt.

22. Verfahren zur Trennung von supergeknäulter Plasmid-DNA von entspannter Plasmid-DNA, umfassend Kontaktieren eines Gemischs aus supergeknäulter Plasmid-DNA und entspannter Plasmid-DNA mit einem hydrophoben Wechselwirkungsmedium unter einer ersten Bedingung, bei der sowohl die supergeknäulte Plasmid-DNA als auch die entspannte Plasmid-DNA an das hydrophobe Wechselwirkungsmedium binden, um ein gebundenes erstes Gemisch zu bilden, Ändern der ersten Bedingung, die das gebundene erste Gemisch umgibt, zu einer zweiten Bedingung, um die entspannte Plasmid-DNA aus dem gebundenen ersten Gemisch zu entfernen, um separate Komponenten zu bilden, die ein zweites gebundenes Gemisch und die entspannte Plasmid-DNA enthalten, sowie Modifizieren der zweiten Bedingung, die das zweite gebundene Gemisch umgibt, zu einer dritten Bedingung, um die supergeknäulte Plasmid-DNA aus dem zweiten gebundenen Gemisch zu entfernen, um separate Komponenten zu bilden, die das hydrophobe Wechselwirkungsmedium und die supergeknäulte Plasmid-DNA enthalten;
wobei das Verfahren in Abwesenheit von organischen Lösungsmitteln, Detergenzien, Glycolen, Hexaminkobalt, Spermidin und Polyvinylpyrrolidon durchgeführt wird.

23. Verfahren nach Anspruch 22, worin die erste Bedingung Äquilibrieren des Mediums mit einer Salzlösung umfasst, die Ammoniumsulfat in einem Konzentrationsbereich von 2,5 M bis 4 M umfasst.

24. Verfahren nach Anspruch 23, worin die zweite Bedingung Waschen der gebundenen ersten Mischung mit einer Salzlösung umfasst, die Ammoniumsulfat in einer Konzentration von 2,35 M bis 2,45 M enthält.

25. Verfahren nach Anspruch 24, worin die dritte Bedingung Waschen des zweiten gebundenen Gemischs mit einer Salzlösung umfasst, die Ammoniumsulfat in einer Konzentration von 1 M bis 2,3 M enthält.

26. Verfahren nach einem der Ansprüche 9 bis 22, worin das Verändern und das Modifizieren in einem kontinuierlichen Vorgang kombiniert werden, umfassend die Gradientenelution der entspannten Plasmid-DNA und der supergeknäulten Plasmid-DNA durch Mischen des gebundenen ersten Gemischs mit einer Ammoniumsulfat enthaltenden Salzlösung mit einer kontinuierlich variierenden Konzentration an Ammoniumsulfat, wobei die Konzentration von 3 M bis 1 M Ammoniumsulfat variiert, und die entspannte Plasmid-DNA in einer ersten eluierten Volumeneinheit gesammelt wird und die supergeknäulte Plasmid-DNA in einer zweiten eluierten Volumeneinheit gesammelt wird.

27. Verfahren nach Anspruch 22, worin die separate entspannte Plasmid-DNA-Komponente und die separate supergeknäulte Plasmid-DNA-Komponente gesammelt und isoliert werden.

28. Verfahren zur Anreicherung der Menge an supergeknäulter Plasmid-DNA im Verhältnis zu entspannter Plasmid-DNA in einem Gemisch davon, wobei das Verfahren umfasst:
(1) Wechselwirken lassen des Gemischs, das supergeknäulte Plasmid-DNA und entspannte Plasmid-DNA enthält, mit einem hydrophoben Wechselwirkungsmedium, umfassend eine Alkylgruppierung, unter ionischen Bedingungen, wobei die supergeknäulte Plasmid-DNA bevorzugt an das hydrophobe Wechselwirkungsmedium bindet;
(2) Behandeln des hydrophoben Wechselwirkungsmediums, enthaltend die entspannte und supergeknäulte Plasmid-DNA, unter ionischen Bedingungen, die die präferentielle Entfernung der entspannten Plasmid-DNA ermöglichen; und
(3) Eluieren der supergeknäulte Plasmid-DNA aus dem hydrophoben Wechselwirkungsmedium;
wobei das Verfahren in Abwesenheit von organischen Lösungsmitteln, Detergenzien, Glycolen, Hexaminkobalt, Spermidin und Polyvinylpyrrolidon durchgeführt wird.

29. Verfahren zur Entfernung von Lipopolysaccharid (LPS) aus einer Zusammensetzung, die Plasmid-DNA enthält, wobei das Verfahren umfasst:
(1) Wechselwirken lassen des Gemischs, das Plasmid-DNA und LPS enthält, mit einem hydrophoben Wechselwirkungsmedium, umfassend eine Alkylgruppierung, wobei das Wechselwirken unter ionischen Bedingungen erfolgt, bei denen das LPS im Vergleich mit der Plasmid-DNA bevorzugt an das hydrophobe Wechselwirkungsmedium bindet; und
(2) Behandeln des hydrophoben Wechselwirkungsmediums, enthaltend die Plasmid-DNA und das LPS, unter ionischen Bedingungen, die die selektive Entfernung der Plasmid-DNA ermöglichen,
wobei das Verfahren in Abwesenheit von organischen Lösungsmitteln, Detergenzien, Glycolen, Hexaminkobalt, Spermidin und Polyvinylpyrrolidon durchgeführt wird.

30. Verfahren nach einem der Ansprüche 1, 9, 17 oder 22, worin das hydrophobe Wechselwirkungsmedium einen Chromatographieträger mit daran gebundenen hydrophoben Gruppen umfasst.

31. Verfahren nach Anspruch 30, worin die gebundenen Gruppen aus der aus C₃-C₁₀-Alkylgruppen bestehenden Gruppe ausgewählt sind.

32. Verfahren nach Anspruch 30 in Abhängigkeit von den Ansprüchen 9 oder 17, worin das hydrophobe Wechselwirkungsmedium aus der aus einer Methacrylat-Polymer- oder -Copolymer-Hauptkette, gebunden an zumindest eines von einem Propyl, Butyl, Hexyl, Octyl, Nonyl oder einem Gemisch dieser als daran gebundene hydrophobe Gruppen, bestehenden Gruppe ausgewählt ist.

33. Verfahren nach Anspruch 30 in Abhängigkeit von Anspruch 1, worin das hydrophobe Wechselwirkungsmedium aus der aus einer Methacrylat-Polymer - oder -Copolymer-Hauptkette, gebunden an zumindest eine von einer hydrophoben Propyl, Butyl, Hexyl, Octyl, Nonyl oder Decyl-Gruppe, bestehenden Gruppe ausgewählt ist.

34. Verfahren nach Anspruch 30 in Abhängigkeit von Anspruch 22, worin das hydrophobe Wechselwirkungsmedium eine Methacrylat-Polymer- oder -Copolymer-Hauptkette, gebunden an zumindest einen von einem Propyl, Butyl, Pentyl, Hexyl, Heptyl, Octyl, Nonyl oder Decyl-Liganden, ist.

35. Verfahren nach Anspruch 30, worin das Medium zumindest eines von einer Methacrylat-Ethylenglycol-Copolymer-Hauptkette oder einer vernetzten Agarose ist.

36. Verfahren nach Anspruch 30, worin das Medium ein Harz in Form von Perlen im Größenbereich von 15 bis 100 µm ist.

37. Verfahren nach Anspruch 35, worin das Salz Ammoniumsulfat in einem Konzentrationsbereich von 2,5 M bis 4 M ist.

## Revendications

1. Procédé pour purifier un ADN plasmidique à partir d'un mélange de celui-ci contenant au moins une impureté de cellule hôte, comprenant les étapes suivantes:
(a) former une solution en ajoutant suffisamment de sel audit mélange, pour permettre une liaison sélective de ladite au moins une impureté de cellule hôte à un milieu d'interaction hydrophobe, où ledit sel est l'ammonium sulfate ou des sels de sodium dans une plage de concentration de 2 M à 4 M;
(b) mettre en contact ladite solution contenant l'ADN plasmidique avec ledit milieu d'interaction hydrophobe dans des conditions qui font que ladite au moins une impureté se lie au milieu d'interaction hydrophobe pour former un complexe; et
(c) collecter l'ADN plasmidique non lié à partir dudit complexe comme écoulement traversant du processus de charge;
où ledit procédé est réalisé en l'absence de solvants organiques, de détergents, de glycols, de cobalt hexamine, de spermidine et de polyvinylpyrrolidone.

2. Procédé selon la revendication 1, où la au moins une impureté est sélectionnée dans le groupe consistant en de l'ARN, une endotoxine, un ADN chromosomique et une protéine.

3. Procédé selon la revendication 1, où la au moins une impureté est une endotoxine.

4. Procédé selon la revendication 1, où le sulfate d'ammonium est présent à une concentration de 2 M.

5. Procédé selon la revendication 1, où le sel de sodium est du chlorure de sodium.

6. Procédé selon la revendication 5, où le sel de sodium est du chlorure de sodium à une concentration de 2 M.

7. Procédé selon la revendication 1, où la solution possède un pH compris dans la plage de 6,8 à 7,4.

8. Procédé selon la revendication 1, où la solution possède un pH de 7,4.

9. Procédé pour séparer un ADN plasmidique surenroulé d'un mélange d'ADN plasmidique surenroulé et d'ADN plasmidique relâché et, facultativement, au moins une impureté de cellule hôte comprenant les étapes suivantes:
(a) former une solution en ajoutant un sel au mélange d'ADN plasmidique surenroulé et d'ADN plasmidique relâché et, lorsqu'elle est présente, à ladite au moins une impureté de cellule hôte;
(b) mettre en contact la solution avec un milieu d'interaction hydrophobe dans une première condition où à la fois l'ADN plasmidique surenroulé et l'ADN plasmidique relâché se lient au milieu d'interaction hydrophobe pour former un premier mélange lié;
(c) altérer la première condition entourant le premier mélange lié en une deuxième condition pour éliminer l'ADN plasmidique relâché du premier mélange lié afin de former des composants séparés contenant un deuxième mélange lié et de l'ADN plasmidique relâché; et
(d) modifier la deuxième condition entourant ledit deuxième mélange lié en une troisième condition pour éliminer l'ADN plasmidique surenroulé dudit deuxième mélange lié afin de former des composants séparés contenant un milieu d'interaction hydrophobe et de l'ADN plasmidique surenroulé, où le procédé est réalisé en l'absence de solvants organiques, de détergents, de glycols, de cobalt hexamine, de spermidine et de polyvinylpyrrolidone.

10. Procédé selon la revendication 9, où la au moins une impureté de cellule hôte est sélectionnée dans le groupe consistant en de l'ARN, une endotoxine, un ADN chromosomique et une protéine.

11. Procédé selon la revendication 9, où la au moins une impureté de cellule hôte est une endotoxine.

12. Procédé selon la revendication 9, où le sel comprend un anion ou un cation sélectionné dans le groupe consistant en SO₄²⁻, Cl⁻, Br⁻, NO₃⁻, Mg²⁺, Li⁺, + Na⁺, K⁺ et NH₄⁺.

13. Procédé selon la revendication 12, où le sel est du sulfate d'ammonium dans une plage de concentration comprise entre 2,5 M et 4 M.

14. Procédé selon la revendication 9, où la première condition consiste à équilibrer ledit milieu avec une solution saline contenant du sulfate d'ammonium qui est présent dans une plage de concentration comprise entre 2,5 M et 4 M.

15. Procédé selon la revendication 9, où la deuxième condition consiste à laver le milieu avec une solution saline contenant du sulfate d'ammonium à une concentration de 2,35 M à 2,45 M.

16. Procédé selon la revendication 9, où ladite troisième condition consiste à laver ledit deuxième milieu lié avec une solution saline contenant du sulfate d'ammonium à une concentration comprise entre 1 M et 2,3 M.

17. Procédé pour séparer une endotoxine d'un ADN plasmidique, consistant à mettre en contact un mélange d'endotoxine et d'ADN plasmidique avec un milieu d'interaction hydrophobe dans des conditions où ladite endotoxine se lie audit milieu d'interaction hydrophobe afin de former un complexe, et à séparer ledit ADN plasmidique et ledit complexe en collectant ledit ADN plasmidique comme écoulement traversant du processus de charge, où ledit procédé est réalisé en l'absence de solvants organiques, de détergents, de glycols, de cobalt hexamine, de spermidine, et de polyvinylpyrrolidone.

18. Procédé selon la revendication 17, où ledit mélange comprend en outre un sel d'ammonium dans une plage de concentration comprise entre 1,5 et 4 M.

19. Procédé selon la revendication 18, où ledit sel d'ammonium est du sulfate d'ammonium qui est présent à une concentration de 2 M.

20. Procédé selon la revendication 17, où ledit mélange possède un pH compris dans la plage de 6,8 à 7,4.

21. Procédé selon la revendication 20, où le pH est de 7,4.

22. Procédé pour séparer un ADN plasmidique surenroulé d'un ADN plasmidique relâché, consistant à mettre en contact un mélange d'ADN plasmidique surenroulé et d'ADN plasmidique relâché avec un milieu d'interaction hydrophobe dans une première condition où à la fois l'ADN plasmidique surenroulé et l'ADN plasmidique relâché se lient audit milieu d'interaction hydrophobe afin de former un premier mélange lié, altérer ladite première condition entourant le premier mélange lié en une deuxième condition pour éliminer ledit ADN plasmidique relâché dudit premier mélange lié afin de former des composants séparés contenant un deuxième mélange lié et ledit ADN plasmidique relâché, et modifier la deuxième condition entourant ledit deuxième mélange lié en une troisième condition pour éliminer ledit ADN plasmidique surenroulé dudit deuxième mélange lié afin de former des composants séparés contenant ledit milieu d'interaction hydrophobe et ledit ADN plasmidique surenroulé;
où ledit procédé est réalisé en l'absence de solvants organiques, de détergents, de glycols, de cobalt hexamine, de spermidine, et de polyvinylpyrrolidone.

23. Procédé selon la revendication 22, où ladite première condition consiste à équilibrer ledit milieu avec une solution saline contenant du sulfate d'ammonium dans une plage de concentration comprise entre 2,5 M et 4 M.

24. Procédé selon la revendication 23, où ladite deuxième condition consiste à laver ledit premier mélange lié avec une solution saline contenant du sulfate d'ammonium à une concentration comprise entre 2,35 M et 2,45 M.

25. Procédé selon la revendication 24, où ladite troisième condition consiste à laver ledit deuxième mélange lié avec une solution saline contenant du sulfate d'ammonium à une concentration comprise entre 1 M et 2,3 M.

26. Procédé selon l'une quelconque des revendications 9 et 22, où ladite altération et ladite modification sont combinées dans un processus continu comprenant une élution par gradient desdits ADN plasmidique relâché et ADN plasmidique surenroulé en mélangeant ledit premier mélange lié avec une solution saline contenant du sulfate d'ammonium ayant une concentration de sulfate d'ammonium continuellement variable, ladite concentration variant entre 3 M et 1 M de sulfate d'ammonium, et ledit ADN plasmidique relâché est collecté dans un premier volume élué et ledit ADN plasmidique surenroulé est collecté dans un second volume élué.

27. Procédé selon la revendication 22, où ledit composant d'ADN plasmidique relâché séparé et ledit ADN plasmidique surenroulé séparé sont collectés et isolés.

28. Procédé pour enrichir la quantité d'ADN surenroulé par rapport à l'ADN relâché dans un mélange de ceux-ci, le procédé consistant à:
(1) faire interagir le mélange contenant l'ADN plasmidique surenroulé et l'ADN plasmidique relâché avec un milieu d'interaction hydrophobe comprenant un fragment alkyle dans des conditions ioniques, où l'ADN plasmidique surenroulé se lie préférentiellement au milieu d'interaction hydrophobe;
(2) traiter le milieu d'interaction hydrophobe contenant l'ADN plasmidique relâché et surenroulé dans des conditions ioniques qui permettent l'élimination préférentielle de l'ADN plasmidique relâché; et
(3) éluer l'ADN plasmidique surenroulé du milieu d'interaction hydrophobe;
où ledit procédé est réalisé en l'absence de solvants organiques, de détergents, de glycols, de cobalt hexamine, de spermidine, et de polyvinylpyrrolidone.

29. Procédé pour éliminer un lipopolysaccharide (LPS) d'une composition contenant de l'ADN plasmidique, le procédé consistant à:
(1) faire interagir le mélange comprenant l'ADN plasmidique et le LPS avec un milieu d'interaction hydrophobe comprenant un fragment alkyle, où l'interaction est soumise à des conditions ioniques dans lesquelles le LPS se lie préférentiellement au milieu d'interaction hydrophobe par rapport à l'ADN plasmidique; et
(2) traiter le milieu d'interaction hydrophobe contenant l'ADN plasmidique et le LPS avec des conditions ioniques qui permettent l'élimination sélective de l'ADN,
où le procédé est réalisé en l'absence de solvants organiques, de détergents, de glycols, de cobalt hexamine, de spermidine et de polyvinylpyrrolidone.

30. Procédé selon l'une quelconque des revendications 1, 9, 17 ou 22, où le milieu d'interaction hydrophobe comprend un support de chromatographie ayant des groupes latéraux hydrophobes.

31. Procédé selon la revendication 30, où lesdits groupes latéraux sont sélectionnés dans le groupe consistant en des groupes alkyles C₃ à C₁₀.

32. Procédé selon la revendication 30 par rapport aux revendications 9 ou 17, où le milieu d'interaction hydrophobe est sélectionné dans le groupe consistant en un squelette de polymère ou de copolymère de méthacrylate lié à au moins l'un de propyle, butyle, hexyle, octyle, nonyle ou un mélange de ceux-ci en tant que groupes latéraux hydrophobes.

33. Procédé selon la revendication 30 par rapport à la revendication 1, où le milieu d'interaction hydrophobe est sélectionné dans le groupe consistant en un squelette de polymère ou de copolymère de méthacrylate lié à au moins l'un des groupes hydrophobes propyle, butyle, hexyle, octyle, nonyle ou décyle.

34. Procédé selon la revendication 30 par rapport à la revendication 22, où le milieu d'interaction hydrophobe est un squelette de polymère ou de copolymère de méthacrylate lié à au moins l'un d'un ligand propyle, butyle, pentyle, hexyle, heptyle, octyle, nonyle ou décyle.

35. Procédé selon la revendication 30, où le milieu est au moins l'un d'un squelette de copolymère de méthacrylate éthylène glycol ou d'agarose réticulée.

36. Procédé selon la revendication 30, où le milieu est une résine sous la forme de billes ayant une taille comprise dans la plage de 15 à 100 µm.

37. Procédé selon la revendication 35, où le sel est du sulfate d'ammonium dans une plage de concentration comprise entre 2,5 M et 4 M.
